(19) Europäisches Patentamt
European Patent Office
Office européen des brevets

(11) EP 0 970 059 B1

(12) **EUROPÄISCHE PATENTSCHRIFT**

(45) Veröffentlichungstag und Bekanntmachung des Hinweises auf die Patenterteilung:
**20.08.2003 Patentblatt 2003/34**

(21) Anmeldenummer: **98912346.8**

(22) Anmeldetag: **20.02.1998**

(51) Int Cl.[7]: **C07D 263/14**, A01N 43/76, C07C 233/73

(86) Internationale Anmeldenummer:
**PCT/EP98/00973**

(87) Internationale Veröffentlichungsnummer:
**WO 98/039310 (11.09.1998 Gazette 1998/36)**

(54) **DISUBSTITUIERTE BIPHENYLOXAZOLINE**
DISUBSTITUTED BIPHENYLOXAZOLINES
BIPHENYLOXAZOLINES DISUBSTITUEES

(84) Benannte Vertragsstaaten:
**AT BE CH DE DK ES FR GB IT LI NL**

(30) Priorität: **05.03.1997 DE 19708923**
**02.05.1997 DE 19718522**
**22.12.1997 DE 19757223**

(43) Veröffentlichungstag der Anmeldung:
**12.01.2000 Patentblatt 2000/02**

(73) Patentinhaber: **Bayer CropScience AG**
**40789 Monheim (DE)**

(72) Erfinder:
- **KRAATZ, Udo**
  **D-51375 Leverkusen (DE)**
- **KRÄMER, Wolfgang**
  **D-51399 Burscheid (DE)**
- **MARHOLD, Albrecht**
  **D-51373 Leverkusen (DE)**
- **ERDELEN, Christoph**
  **D-42799 Leichlingen (DE)**
- **WACHENDORFF-NEUMANN, Ulrike**
  **D-56566 Neuwied (DE)**
- **TURBERG, Andreas**
  **D-42781 Haan (DE)**
- **MENCKE, Norbert**
  **D-51381 Leverkusen (DE)**

(56) Entgegenhaltungen:
**EP-A- 0 432 661** **EP-A- 0 645 085**
**EP-A- 0 696 584** **WO-A-96/22283**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft neue Biphenyloxazoline, Verfahren zu ihrer Herstellung und die Verwendung der Biphenyloxazoline zur Bekämpfung von tierischen Schädlingen.

Es ist bekannt, daß bestimmte Biphenyloxazoline insektizid und akarizid wirksam sind, beispielsweise die Verbindungen 2-(2,6-Difluorphenyl)-4-(3'-chlor-4'-(1,1,2,2-tetrafluorethoxy)-biphenyl-4)-2-oxazolin (EP-A-0 696 584) und 2-(2,6-Difluorphenyl)-4-(3'-chlor-4'-methylbiphenyl-4)-2-oxazolin (EP-A-0 432 661).

Weiterhin sind aus EP 0 645 085 und WO 96/22283 substituierte Oxazoline und Thiazoline bekannt, die akarizid und/oder insektizid wirksam sind.

Die Wirkungshöhe und/oder Wirkungsdauer dieser bekannten Verbindungen ist jedoch, insbesondere gegen bestimmte Organismen oder bei niedrigen Anwendungskonzentrationen, nicht in allen Anwendungsgebieten völlig zufriedenstellend.

Es wurden neue disubstituierte Biphenyloxazoline der Formel (I)

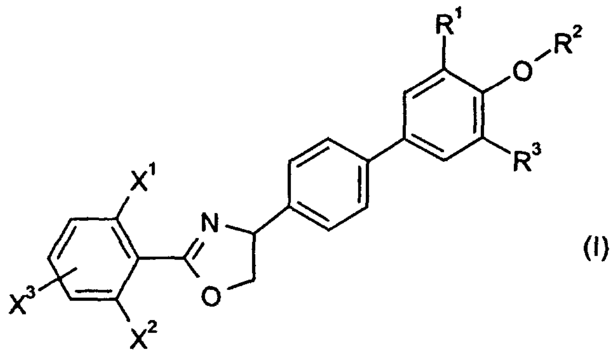

(I)

gefunden,
in welcher

$X^1$ für Wasserstoff, Fluor, Chlor, Brom, Iod, $C_1$-$C_6$-Alkyl, $C_1$-$C_6$-Alkoxy oder $C_1$-$C_6$-Alkylthio.

$X^2$ für Fluor, Chlor, Brom, Iod, $C_1$-$C_6$-Alkyl, $C_1$-$C_6$-Alkoxy oder $C_1$-$C_6$-Alkylthio.

$X^3$ für Wasserstoff, Fluor, Chlor, $C_1$-$C_6$-Alkyl oder $C_1$-$C_6$-Alkoxy, insbesondere für Wasserstoff.

$R^1$ für Wasserstoff, Fluor, Chlor, Brom, $C_1$-$C_6$-Alkyl oder $-CH_2-CR^4=CH_2$.

$R^2$ für $C_1$-$C_8$-Alkyl, $C_1$-$C_6$-Hydroxyalkyl, $C_3$-$C_{10}$-Alkenyl, $C_3$-$C_{12}$-Alkinyl, für jeweils gegebenenfalls durch Halogen oder $C_1$-$C_4$-Alkyl substituiertes $C_3$-$C_6$-Cycloalkyl oder $C_4$-$C_6$-Cycloalkenyl,
für gegebenenfalls durch Halogen, $C_1$-$C_4$-Alkyl, $C_2$-$C_4$-Alkenyl, $C_2$-$C_4$-Halogenalkenyl, Phenyl, Halogenphenyl, Styryl oder Halogenstyryl sub-stituiertes $C_3$-$C_6$-Cycloalkyl-$C_1$-$C_4$-alkyl,
für gegebenenfalls durch Halogen oder $C_1$-$C_4$-Alkyl substituiertes $C_4$-$C_8$-Cycloalkenyl-$C_1$-$C_2$-alkyl,
oder für gegebenenfalls einfach bis vierfach, gleich oder verschieden durch Nitro, Halogen, $C_1$-$C_{12}$-Alkyl, $C_1$-$C_{12}$-Halogenalkyl, $C_1$-$C_{12}$-Alkylthio, $C_1$-$C_{12}$-Halogenalkylthio, $C_1$-$C_{12}$-Alkoxy oder $C_1$-$C_{12}$-Halogenalkoxy sub-stituiertes Phenyl-$C_1$-$C_4$-alkyl, für Naphthyl-$C_1$-$C_3$-alkyl oder Tetrahydro-naphthyl-$C_1$-$C_3$-alkyl.

$R^3$ für Wasserstoff, $C_1$-$C_4$-Alkyl, Fluor, Chlor, Brom oder für $-CH_2-CR^4=CH_2$, wobei $R^1$ und $R^3$ nicht gleichzeitig für Wasserstoff stehen, und

$R^4$ für Wasserstoff, $C_1$-$C_{12}$-Alkyl oder
für gegebenenfalls einfach bis vierfach, gleich oder verschieden durch Halogen, $C_1$-$C_{12}$-Alkyl, $C_1$-$C_{12}$-Halogenalkyl, $C_1$-$C_{12}$-Alkoxy oder $C_1$-$C_{12}$-Halogenalkoxy substituiertes Phenyl steht.

Aufgrund eines oder mehrerer Chiralitätszentren fallen die Verbindungen der Formel (I) im allgemeinen als Stereoisomerengemische an. Sie können sowohl in Form ihrer Diastereomerengemische als auch als reine Diastereomere oder Enantiomere verwendet werden.

Weiter wurde gefunden, daß man die neuen Verbindungen der Formel (I) erhält, wenn man

A) Verbindungen der Formel (II)

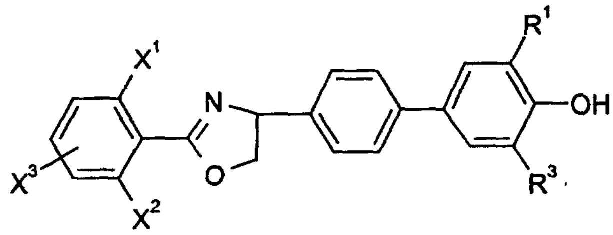

(II),

in welcher
$X^1$, $X^2$, $X^3$, $R^3$ und $R^1$ die oben angegebenen Bedeutungen haben, wobei hier auch $R^3 = R^1$ = Wasserstoff sein darf, wenn $R^2$ für Allyl steht und Schritt B) ausgeführt wird,
mit Verbindungen der Formel (III)

$$Z - R^2 \quad \text{(III)},$$

in welcher

$R^2$ die oben angegebene Bedeutung hat und

Z für eine Abgangsgruppe steht,

gegebenenfalls in Gegenwart eines Verdünnungsmittels und gegebenenfalls in Gegenwart einer Base umsetzt und

B) gegebenenfalls anschließend die so für $R^2 = -CH_2-CR^4=CH_2$ und $R^3$ = H erhaltenen Verbindungen der Formel (IV)

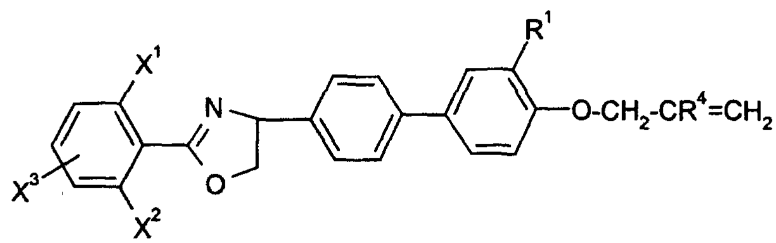

(IV),

in welcher
$R^1$, $R^4$, $X^1$, $X^2$ und $X^3$ die oben angegebenen Bedeutungen haben,
einer Claisen-Umlagerung unterwirft und

C) gegebenenfalls anschließend die so erhaltenen Verbindungen der Formel (V)

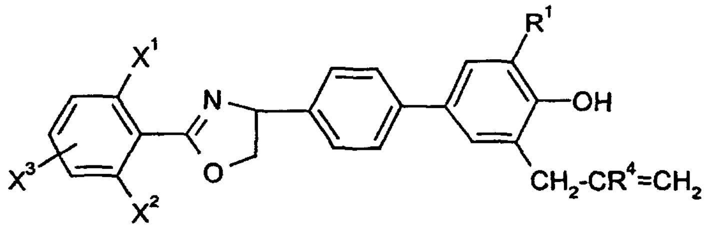

(V),

in welcher
$X^1$, $X^2$, $X^3$, $R^1$ und $R^4$ die oben angegebenen Bedeutungen haben,
mit Verbindungen der Formel (III)

$$Z - R^2 \quad \text{(III)},$$

in welcher
$R^2$ und Z die oben angegebenen Bedeutungen haben,
gegebenenfalls in Gegenwart eines Verdünnungsmittels und gegebenenfalls in Gegenwart einer Base umsetzt und gegebenenfalls anschließend für $R^2$ = $-CH_2-CR^4=CH_2$ und $R^1$ = H die Schritte B) und C) wiederholt, wobei man Verbindungen der Formel (Va) erhält

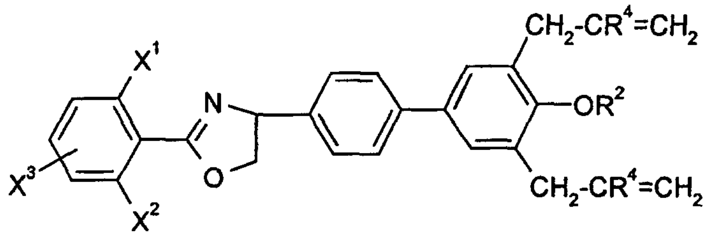

(Va),

in welcher
$X^1$, $X^2$, $X^3$, $R^2$ und $R^4$ die oben angegebene Bedeutung haben,
und die Reste $R^4$ gleich oder verschieden sein können.

Weiter wurde gefunden, daß die neuen Verbindungen der Formel (I) sehr gut zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.
Die erfindungsgemäßen Verbindungen sind durch die Formel (I) allgemein definiert.
Bevorzugte Substituenten bzw. Bereiche der in den oben und nachstehend erwähnten Formeln aufgeführten Reste werden im folgenden erläutert.

$X^1$ steht besonders bevorzugt für Wasserstoff, Fluor oder Chlor.

$X^2$ steht besonders bevorzugt für Fluor, Chlor, Brom, Iod, $C_1$-$C_3$-Alkyl oder $C_1$-$C_3$-Alkoxy.

$X^3$ steht besonders bevorzugt für Wasserstoff, Fluor, Chlor, $C_1$-$C_3$-Alkyl oder $C_1$-$C_3$Alkoxy, insbesondere für Wasserstoff.

$R^1$ steht besonders bevorzugt für Wasserstoff, Fluor, Chlor, Brom, $C_1$-$C_4$-Alkyl oder $-CH_2-CR^4=CH_2$.

$R^2$ steht besonders bevorzugt für $C_1$-$C_6$-Alkyl, $C_1$-$C_4$-Hydroxyalkyl, $C_3$-$C_{10}$-Alkenyl, $C_3$-$C_5$-Alkinyl, für jeweils gegebenenfalls durch Halogen oder $C_1$-$C_4$-Alkyl substituiertes Cyclohexyl oder $C_4$-$C_6$-Cycloalkenyl,
für gegebenenfalls durch Halogen, $C_1$-$C_4$-Alkyl, $C_2$-$C_4$-Alkenyl, $C_2$-$C_3$-Halogenalkenyl, Phenyl, Halogenphenyl, Styryl oder Halogenstyryl substi-tuiertes $C_3$-$C_6$-Cycloalkyl-$C_1$-$C_4$-alkyl,

für gegebenenfalls durch Halogen substituiertes $C_4$-$C_6$-Cycloalkenylmethyl oder
für gegebenenfalls einfach oder zweifach, gleich oder verschieden durch Nitro, Halogen, $C_1$-$C_4$-Alkyl, $C_1$-$C_4$-Halogenalkyl, $C_1$-$C_4$-Alkylthio, $C_1$-$C_4$-Halogenalkylthio, $C_1$-$C_4$-Alkoxy oder $C_1$-$C_4$-Halogenalkoxy substituiertes Benzyl, für Naphthylmethyl oder Tetrahydronaphthylmethyl.

$R^3$ steht besonders bevorzugt für Wasserstoff, $C_1$-$C_3$-Alkyl, Chlor, Brom oder $-CH_2-CR^4=CH_2$, wobei $R^1$ und $R^3$ nicht gleichzeitig für Wasserstoff stehen.

$R^4$ steht besonders bevorzugt für Wasserstoff, $C_1$-$C_4$-Alkyl oder für gegebenen-falls einfach oder zweifach, gleich oder verschieden durch Fluor, Chlor, Brom, $C_1$-$C_4$-Alkyl, $C_1$-$C_4$-Halogenalkyl oder $C_1$-$C_4$-Halogenalkoxy substituiertes Phenyl.
Halogen steht in den als bevorzugt bzw. besonders bevorzugt bezeichneten Restdefinitionen insbesondere für Fluor, Chlor und Brom.

$X^1$ steht ganz besonders bevorzugt für Wasserstoff, Fluor oder Chlor.

$X^2$ steht ganz besonders bevorzugt für Fluor, Chlor, Brom, Iod, Methyl oder Methoxy.

$X^3$ steht ganz besonders bevorzugt für Wasserstoff, Fluor, Chlor oder Methyl, insbesondere für Wasserstoff.

$R^1$ steht ganz besonders bevorzugt für Wasserstoff, Fluor, Chlor, Brom, Methyl, Ethyl, n- oder i-Propyl oder für Allyl.

$R^2$ steht ganz besonders bevorzugt für Methyl, Ethyl, n- oder i-Propyl, n-Butyl, i-Butyl, s-Butyl, n-Pentyl, n-Hexyl, Hydroxyethyl, Hydroxypropyl, Hydroxy-butyl, für $-CH_2-CH=CH_2$, $-CH_2-C(CH_3)=CH_2$, $-CH_2-C\equiv CH$ oder $-CH(CH_3)C\equiv CH$,
für eine der Cycloalkylalkylgruppierungen:

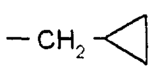 , 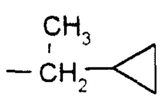 , 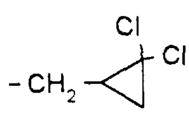 , 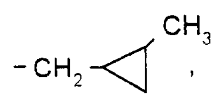 ,

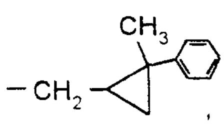 , 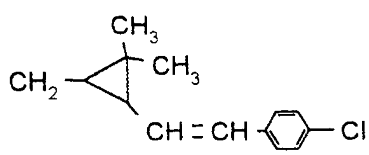 , 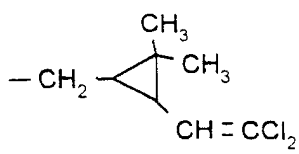 ,

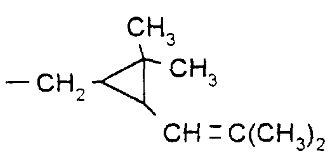 ;

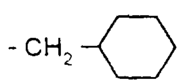 , 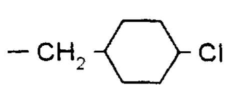 , 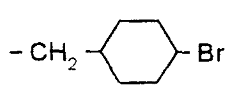 ,

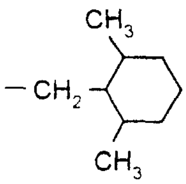 , 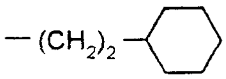 , 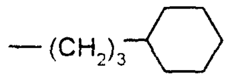 ,

für

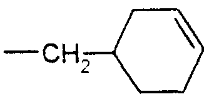 ,

für

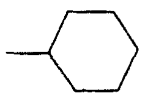 ,

für einen der folgenden Reste:

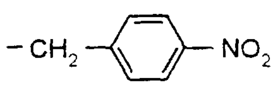 , 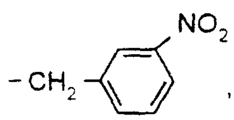 , 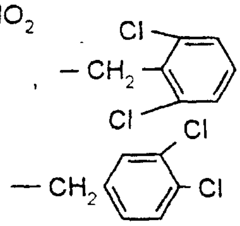 ,

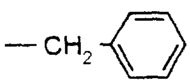 , 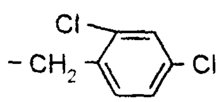 , ,

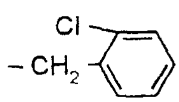 , 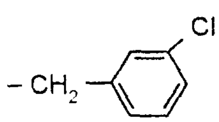 , 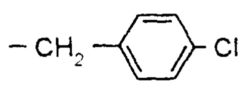 ,

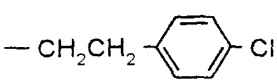 , 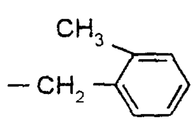 , 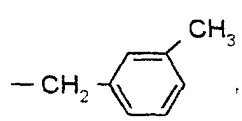 ,

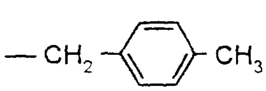 , 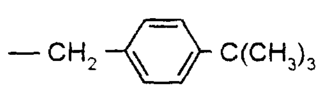 ,

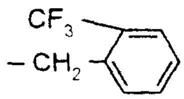, 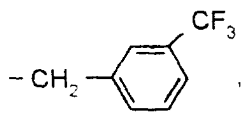, 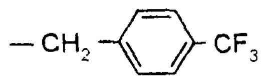,

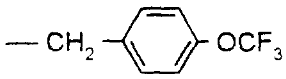, 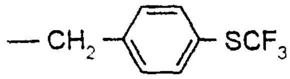, 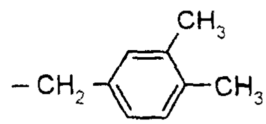,

oder für

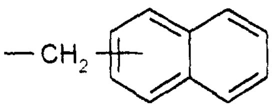

oder

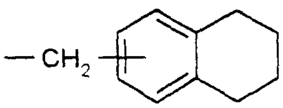.

$R^3$ steht ganz besonders bevorzugt für Wasserstoff, Chlor, Brom, Methyl oder - $CH_2$-CH=$CH_2$, insbesondere für Wasserstoff, Chlor oder -$CH_2$-CH=$CH_2$, wobei $R^1$ und $R^3$ nicht gleichzeitig für Wasserstoff stehen.

Bevorzugt sind weiterhin Verbindungen der Formel (Ia)

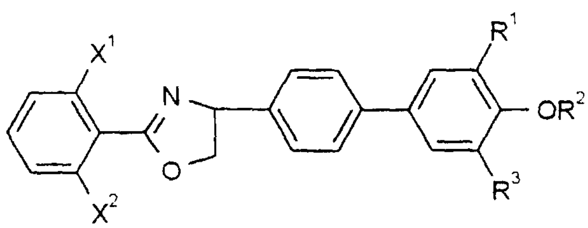

(Ia),

in welcher

$R^1$, $R^2$, $R^3$, $X^1$ und $X^2$ die obengenannten allgemeinen, bevorzugten, besonders bevorzugten oder ganz besonders bevorzugten Bedeutungen haben.

Dabei gilt jeweils, daß $R^1$ und $R^3$ nicht gleichzeitig für Wasserstoff stehen

Eine bevorzugte Gruppe von Verbindungen sind auch solche der Formel (Ib)

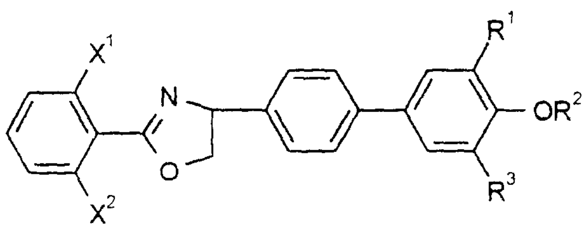

(Ib),

in welcher

$X^1$ für Wasserstoff oder Fluor steht,

$X^2$ für Fluor, Chlor, Brom oder Methyl steht,

$R^1$ für Chlor, Methyl, Ethyl, n- oder i-Propyl oder Allyl steht und

$R^2$ die obengenannten allgemeinen, bevorzugten, besonders bevorzugten und ganz besonders bevorzugten Bedeutungen hat und besonders hervorgehoben für gegebenenfalls wie oben angegeben substituiertes Benzyl steht,

$R^3$ für Wasserstoff, Chlor, Brom, Methyl oder Allyl steht, insbesondere für Wasserstoff, Chlor oder Allyl.

Bevorzugt innerhalb dieser Gruppen sind Verbindungen, in welchen $R^3$ für Wasserstoff steht.

Die oben bei der Definition der erfindungsgemäßen Verbindungen genannten Kohlenwasserstoffreste, wie Alkyl oder Alkenyl, sind - auch in Verbindung mit Heteroatomen, wie in Alkoxy - soweit möglich jeweils geradkettig oder verzweigt.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Verwendet man beispielsweise 2-(2,6-Difluorphenyl)-4-(4'-hydroxy-3'-n-propylbiphenyl-4)-2-oxazolin und Isopropylbromid als Ausgangsstoffe, so läßt sich der Verlauf der Stufe A) des erfindungsgemäßen Verfahrens durch folgendes Reaktionsschema wiedergeben:

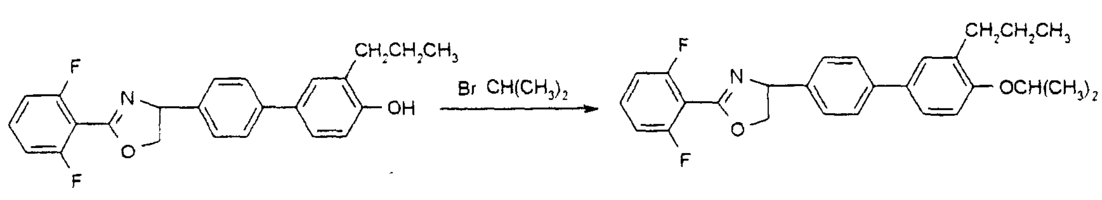


Verwendet man beispielsweise 2-(2,6-Difluorphenyl)-4-(4'-alloxy-3'-chlorbiphenyl-4)-2-oxazolin als Ausgangsstoff, so läßt sich der Verlauf der Stufe B) des erfindungsgemäßen Verfahrens durch folgendes Reaktionsschema wiedergeben:

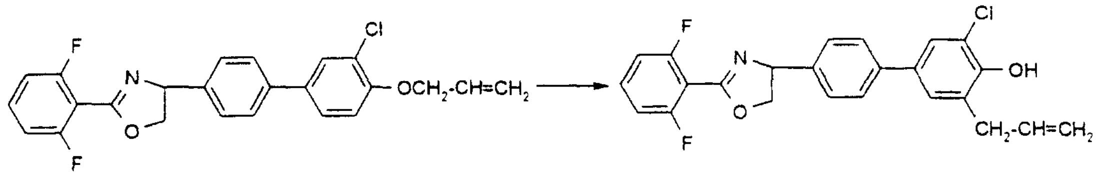

Verwendet man beispielsweise 2-(2,6-Difluorphenyl)-4-(3'-allyl-5'-chlor-4'-hydroxybiphenyl-4)-2-oxazolin und Benzylbromid als Ausgangsstoffe, so läßt sich der Verlauf der Stufe C) des erfindungsgemäßen Verfahrens durch folgendes Reaktionsschema wiedergeben:

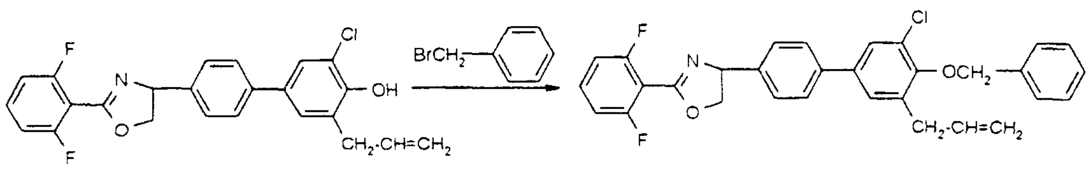

Die bei der Stufe A) des erfindungsgemäßen Verfahrens als Ausgangsstoffe benötigten Verbindungen der Formel (II) sind neu, wenn $R^3$ und $R^1$ nicht gleichzeitig für Wasserstoff stehen.

Man erhält die Verbindungen der Formel (II) beispielsweise, wenn man Verbindungen der Formel (VI)

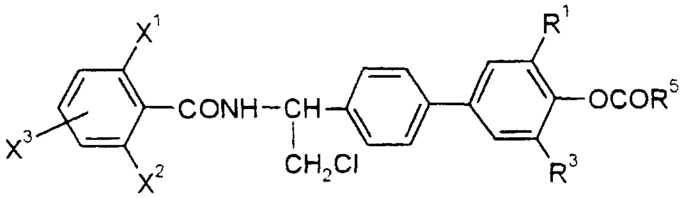

(VI),

in welcher

| | |
|---|---|
| $X^1$, $X^2$, $X^3$, $R^1$ und $R^3$ | die oben angegebenen Bedeutungen haben und |
| $R^5$ | für Alkyl (beispielsweise Methyl), Aryl (beispielsweise Phenyl) oder Alkoxy (beispielsweise Methoxy oder Ethoxy) steht, |

in Gegenwart einer Base, gegebenenfalls in Gegenwart eines Katalysators und gegebenenfalls in Gegenwart eines Verdünnungsmittels cyclisiert und anschließend die Acylgruppe abspaltet (Verfahren D).

Verwendet man beispielsweise 2,6-Difluor-N-[2-chlor-1-(4-(4-acetoxy-3-n-propylphenyl)phenyl)ethyl]-benzamid als Ausgangsstoff, so läßt sich der Verlauf des Verfahrens D) zur Herstellung der Verbindungen der Formel (II) durch folgendes Reaktionsschema wiedergeben:

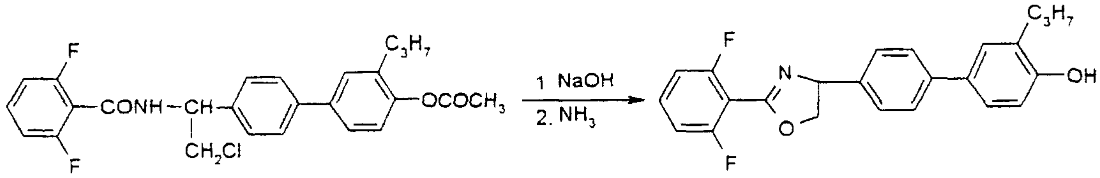


Als Verdünnungsmittel kommen beim Verfahren D) alle inerten organischen Lösungsmittel in Frage. Sie können gegebenenfalls in Mischung mit Wasser verwendet werden. Bevorzugt verwendet werden Kohlenwasserstoffe wie

Toluol, Xylol, Tetralin, Hexan, Cyclohexan, Halogenkohlenwasserstoffe wie Methylenchlorid, Chloroform, Chlorbenzol, o-Dichlorbenzol, Alkohole wie Methanol, Ethanol, Glykol, die isomeren Propanole, Butanole, Pentanole, Ether wie Diethylether, Diisopropylether, Dimethoxyethan, Tetrahydrofuran, Dioxan, Nitrile wie Acetonitril oder Butyronitril, Amide wie Dimethylformamid, Sulfoxide wie Dimethylsulfoxid, ferner Sulfolan. Besonders bevorzugt werden Alkohole oder Dimethylformamid verwendet.

Als Base für die Cyclisierung kommen alle üblichen Säureakzeptoren in Frage.

Vorzugsweise verwendbar sind tertiäre Amine wie Triethylamin, Pyridin, DABCO, DBU, DBN, N,N-Dimethylanilin, ferner Erdalkalimetalloxide wie Magnesium- und Calciumoxid, außerdem Alkali- und Erdalkalimetallcarbonate wie Natriumcarbonat, Kaliumcarbonat und Calciumcarbonat, Alkalihydroxide wie Natrium- und Kaliumhydroxid, ferner Alkoholate wie Natriumethanolat oder Kalium-tert.-butylat.

Gegebenenfalls wird in Gegenwart eines Phasentransferkatalysators gearbeitet. Als Phasentransferkatalysator kommen beispielsweise Ammoniumverbindungen wie Tetraoctylammoniumbromid oder Benzyltriethylammoniumchlorid in Frage.

Die Reaktionstemperatur kann in einem größeren Bereich variiert werden. Im allgemeinen arbeitet man bei Temperaturen zwischen -20°C und 150°C, bevorzugt zwischen -10°C und 100°C.

Die Umsetzung wird im allgemeinen unter Normaldruck durchgeführt

Im allgemeinen wird eine äquimolare Menge an Base eingesetzt. Es ist aber auch möglich, mit einem Basenüberschuß zu arbeiten.

Als Verdünnungsmittel für die anschließende Verseifung werden vorzugsweise Wasser/Alkohol-Gemische verwendet, wie beispielsweise Wasser/Methanol, Wasser/Ethanol oder Wasser/ Propanol oder Wasser/Amidgemische wie beispielsweise Wasser/Dimethylformamid (DMF) oder Wasser/Dimethylacetamid.

Die Verseifung wird in Gegenwart einer Base durchgeführt. Als solche kommen anorganische und organische Basen in Frage, insbesondere Alkalihydroxide wie Natriumhydroxid oder Kaliumhydroxid oder Ammoniak

Die Reaktionstemperatur kann bei der Verseifung in einem größeren Bereich variiert werden. Im allgemeinen arbeitet man bei Temperaturen zwischen -10°C und 60°C, bevorzugt zwischen 0°C und 40°C. Die Umsetzung wird im allgemeinen unter Normaldruck durchgeführt.

Cyclisierung und Abspaltung der Acylgruppen können sowohl als Eintopfreaktion als auch in zwei getrennten Schritten durchgeführt werden.

Die beim Verfahren D) als Ausgangsstoffe benötigten Verbindungen der Formel (VI) sind neu.

Man erhält die Verbindungen der Formel (VI) beispielsweise, wenn man Verbindungen der Formel (VII)

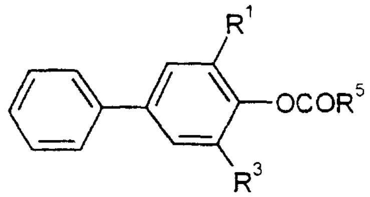

(VII),

in welcher
$R^1$, $R^3$ und $R^5$ die oben angegebenen Bedeutungen haben,
mit Verbindungen der Formel (VIII)

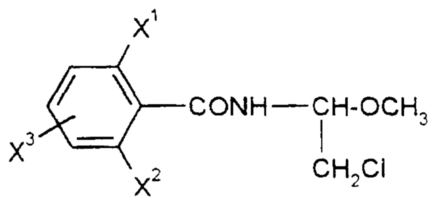

(VIII),

in welcher
$X^1$, $X^2$ und $X^3$ die oben angegebenen Bedeutungen haben,
in Gegenwart eines sauren Katalysators, beispielsweise einer Lewis-Säure wie Eisen(III)-chlorid, Aluminiumchlorid

oder Fluorwasserstoff und bevorzugt in Gegenwart eines Verdünnungsmittels, beispielsweise eines halogenierten, insbesondere chlorierten Kohlenwasserstoffs wie Dichlormethan bei Temperaturen zwischen -20°C und 80°C umsetzt (vgl. die Herstellungsbeispiele und auch WO 96/18619).

Die Verbindungen der Formel (VII) sind teilweise bekannt (J. Amer. Chem. Soc. 61, 1447, 3037 (1939); J. Amer. Chem. Soc. 56, 202 (1934); J. Amer. Chem. Soc. 64, 2219 (1942); J. Amer. Chem. Soc. 89, 2711 (1967); J. Org. Chem. 27, 2671 (1962); J. Indian Chem. Soc. 12, 410 (1935); DE-1 911 520) oder können nach bekannten Methoden hergestellt werden, beispielsweise durch Umsetzung der entsprechenden Hydroxybiphenyle der Formel (IX) mit den Säureanhydriden der Formel (X) oder den Säurehalogeniden (insbesondere Säurechloriden) der Formel (XI):

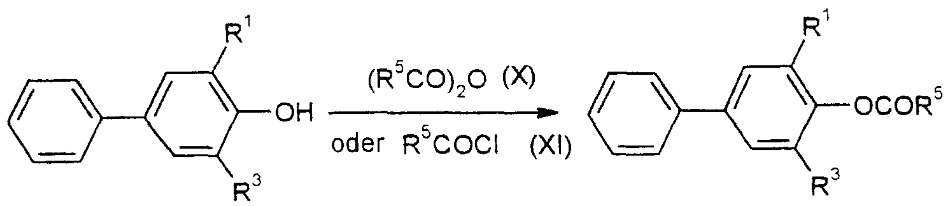


(IX) (VII)

Die Verbindungen der Formel (IX) sind teilweise bekannt (siehe z.B. J.Org.Chem. 29, 2640 (1964), J. Indian Chem. Soc. 12, 410 (1935), J. Amer. Chem. Soc. 80, 3271 (1958), J. Amer. Soc. 56, 202 (1934), J. Org. Chem. 29, 3014 (1964)) oder lassen sich nach bekannten Methoden herstellen (vgl. die Herstellungsbeispiele), z.B. auf folgendem Weg

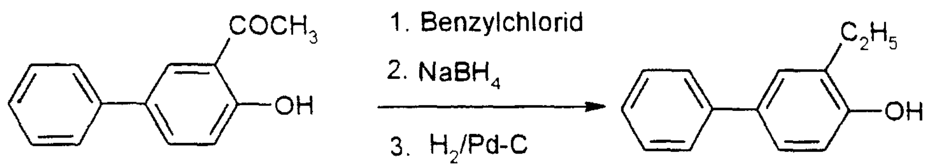


,

wobei die Ausgangsverbindung aus Bull. Chem. Soc. Jap. 56, 2037 (1983) bekannt ist oder gemäß

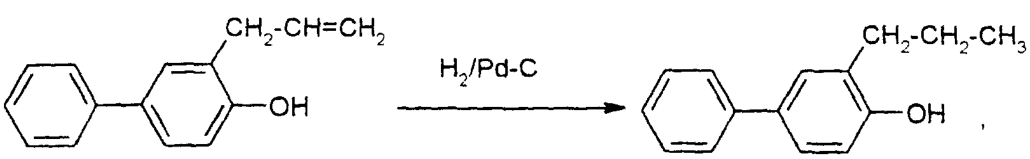


,

wobei die Ausgangsverbindung bekannt ist (siehe J. Amer. Chem. Soc. 80, 3271, (1958)).

Die Verbindungen der Formeln (X) und (XI) sind allgemein bekannt und häufig käuflich oder in einfacher und bekannter Weise aus den entsprechenden Carbonsäuren erhältlich.

Die Verbindungen der Formel (VIII) sind bekannt (siehe z.B. EP-A-0 594 179, WO 96/18619) oder können nach den dort beschriebenen Methoden hergestellt werden.

Die Verbindungen der Formel (VI) können auch erhalten werden, wenn man Säurechloride der Formel (XII).

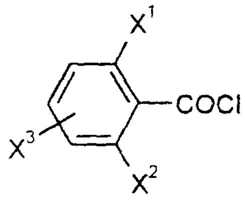

(XII),

in welcher
$X^1$, $X^2$ und $X^3$ die oben angegebenen Bedeutungen haben,
mit Aminoalkoholen der Formel (XIII)

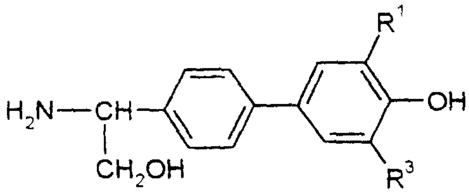

(XIII),

in welcher

$R^1$ und $R^3$ die oben angegebenen Bedeutung haben,

gegebenenfalls in Gegenwart eines Verdünnungsmittels, (als Verdünnungsmittel können alle gegenüber diesen Verbindungen inerten Solventien eingesetzt werden, vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan; wenn die Hydrolysestabilität des Säurehalogenids es zuläßt, kann die Umsetzung auch in Gegenwart von Wasser durchgeführt werden) und gegebenenfalls in Gegenwart einer Base (als Base kommen bei der Umsetzung alle üblichen Säureakzeptoren in Betracht, vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat und Alkali- oder Erdalkalihydroxide wie Natriumhydroxid oder Kaliumhydroxid) bei Temperaturen zwischen -20°C und +100°C, vorzugsweise zwischen 0°C und 30°C umsetzt und gegebenenfalls anschließend die so erhaltenen Verbindungen der Formel (XIV)

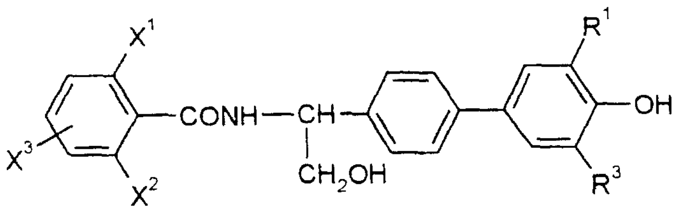

(XIV),

in welcher
$X^1$, $X^2$, $X^3$, $R^3$ und $R^1$ die oben angegebenen Bedeutungen haben,
mit einem Chlorierungsmittel wie Thionylchlorid, Phosgen oder Phosphoroxychlorid, gegebenenfalls in Gegenwart eines Verdünnungsmittels, beispielsweise Kohlenwasserstoffen wie Toluol, Xylol, Hexan, Cyclohexan, Halogenkohlenwasserstoffen wie Chlorbenzol, Chloroform, Methylenchlorid oder Ethern wie Diethylether, Diisopropylether, Dimethoxyethan, Tetrahydrofuran oder Dioxan, bei Temperaturen zwischen 0°C und 120°C, bevorzugt zwischen 20°C und 100°C umsetzt (vgl. EP-A-0 696 584).

Die Verbindungen der Formel (XII) sind allgemein bekannt und häufig käuflich oder in einfacher und bekannter Weise aus den entsprechenden Carbonsäuren erhältlich.

Die Verbindungen (XIII) sind bekannt (vgl. EP-A 0 696 584) oder können nach den dort beschriebenen Methoden hergestellt werden.

Die weiterhin in der Stufe A) des erfindungsgemäßen Verfahrens als Ausgangsstoffe benötigten Verbindungen der Formel (III) sind allgemein bekannte Verbindungen der organischen Chemie. Z steht für eine übliche Abgangsgruppe, beispielsweise für Halogen (insbesondere Chlor oder Brom), Alkylsulfonyloxy (insbesondere Methylsulfonyloxy) oder gegebenenfalls substituiertes Arylsulfonyloxy (insbesondere Phenylsulfonyloxy, p-Chlorphenylsulfonyloxy oder Tolylsulfonyloxy).

Stufe A) des erfindungsgemäßen Verfahrens ist dadurch gekennzeichnet, daß man eine Verbindung der Formel (II) mit einer Verbindung der Formel (III) gegebenenfalls in Gegenwart eines Verdünnungsmittels und gegebenenfalls in Gegenwart einer Base umsetzt.

Als Verdünnungsmittel kommen alle üblichen Lösungsmittel in Frage. Vorzugsweise verwendbar sind gegebenenfalls halogenierte, aromatische oder aliphatische Kohlenwasserstoffe, Ketone, Nitrile und Amide. Genannt seien beispielsweise Toluol, Aceton, Acetonitril, Dimethylformamid und Dimethylacetamid

Als Base kommen alle üblichen anorganischen und organischen Basen in Frage. Genannt seien beispielsweise tertiäre Amine wie Triethylamin, DBN, DBU, DABCO, Alkali- und Erdalkalihydroxide wie z.B. Natriumhydroxid, Kaliumhydroxid und Calciumhydroxid sowie Alkali- und Erdalkalicarbonate wie z.B. Natriumcarbonat oder Kaliumcarbonat.

Die Reaktionstemperatur kann innerhalb eines größeren Bereiches variiert werden. Im allgemeinen arbeitet man bei Temperaturen zwischen -20°C und 100°C, bevorzugt zwischen 0°C und 60°C.

Die Umsetzung wird im allgemeinen unter Normaldruck durchgeführt.

Die Verbindungen der Formel (II) und die Verbindungen der Formel (III) werden im allgemeinen in angenähert äquimolaren Mengen eingesetzt. Man kann jedoch auch einen Überschuß der Verbindungen der Formel (III) verwenden.

Stufe B) des erfindungsgemäßen Verfahrens ist dadurch gekennzeichnet, daß man Verbindungen der Formel (IV) einer Claisen-Umlagerung unterwirft.

Hierbei werden die Verbindungen der Formel (IV) in Substanz oder in Gegenwart eines Verdünnungsmittels (Chlorbenzol, Dichlorbenzol, Diethylanilin) auf Temperaturen von 160°C bis 290°C, bevorzugt 180°C bis 260°C erhitzt (siehe auch D.S. Tarbell, The Claisen Rearrangement, Org. Reactions 2, 1ff (1944)).

Stufe C) des erfindungsgemäßen Verfahrens ist dadurch gekennzeichnet, daß man Verbindungen der Formel (V) mit Verbindungen der Formel (III), gegebenenfalls in Gegenwart eines Verdünnungsmittels und gegebenenfalls in Gegenwart einer Base umsetzt.

Für diese zur Stufe A) analoge Umsetzung gilt hinsichtlich Verdünnungsmittel, Base, Reaktionstemperatur etc. das dort gesagte.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.,

Aus der Ordnung der Anoplura z.B. Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipuia paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.,

Die erfindungsgemäßen Verbindungen der Formel (1) zeichnen sich insbesondere durch hervorragende insektizide und akarizide Wirksamkeit aus.

Sie lassen sich mit besonders gutem Erfolg zur Bekämpfung von pflanzenschädigenden Insekten, wie beispielsweise gegen die Meerrettichblattkäfer-Larven (Phaedon cochleariae), die Raupen des Eulenfalters (Spodoptera frugiperda) und die Pfirsichblattläuse (Myzus persicae) oder zur Bekämpfung von pflanzenschädigenden Milben, wie

beispielsweise gegen die gemeine Spinnmilbe (Tetranychus urticae) einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 % und bevorzugt daneben Streckmittel und/oder oberflächenaktive Mittel.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

**Fungizide:**

2-Aminobutan, 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-DichloroN-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino[alpha-(otolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,

Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon, Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

**Bakterizide:**

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide:**

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin,
Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301/5302, Zetamethrin.
Auch eine Mischung mit anderen bekannten Wirkstoffen, wie Herbiziden oder mit Düngemitteln und Wachstumsregulatoren ist möglich.
Die erfindungsgemäßen Wirkstoffe können ferner in ihren handelsüblichen Formulierungen sowie in den aus diesen

Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.

Aus der Ordnung der Actinedida (Prostigmata) und Acaridida (Astigmata) z.B Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine gute entwicklungshemmende Wirkung gegen Fliegenlarven von Lucilia cuprina.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis; Xyleborus spec. Tryptodendron spec. Apate monachus, Bostrychus capucins, Heterobostrychus brunneus, Sinoxylon spec. Dinoderus minutus.

Hautflügler wie

Sirex juvencus, Urocerus gigas, Urocerus gigas taignus, Urocerus augur.

Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze wie

Lepisma saccarina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden können, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und- türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige

schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dergleichen zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindelöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dergleichen eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. dem Ausfällen vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlofluanid, Tolylfluanid, 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe gehen aus den nachfolgenden Beispielen hervor.

**Herstellungsbeispiele**

**Beispiel (I-1)**

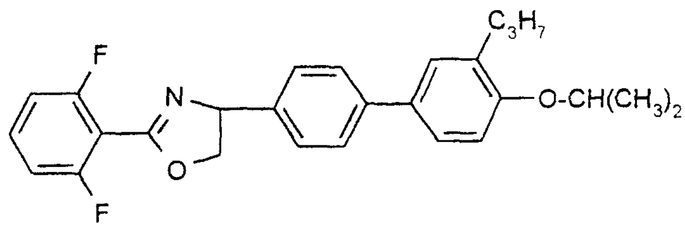

2-(2,6-Difluorphenyl)-4-(4-(4-propyl-2-oxy)-3-n-propylphenyl)phenyl-2-oxazolin: 1,5 g (3,8 mMol) 2-(2,6-Difluorphenyl)-4-(4-(4-hydroxy-3-n-propylphenyl)phenyl)-2-oxazolin werden in 30 ml Acetonitril mit 0,6 g (4,5 mMol) Kaliumcarbonat und 6,0 g (48 mMol) 2-Brompropan 10 Stunden unter Rückfluß gekocht. Man gibt dann auf Wasser und extrahiert das Produkt mit Essigester. Die organische Phase wird getrocknet, im Vakuum eingeengt und der Rückstand an Kieselgel im System Cyclohexan/Essigester (2:1) gereinigt.
Ausbeute: 0,45 g; Fp. 60-62°C.

**Beispiel (I-342)**

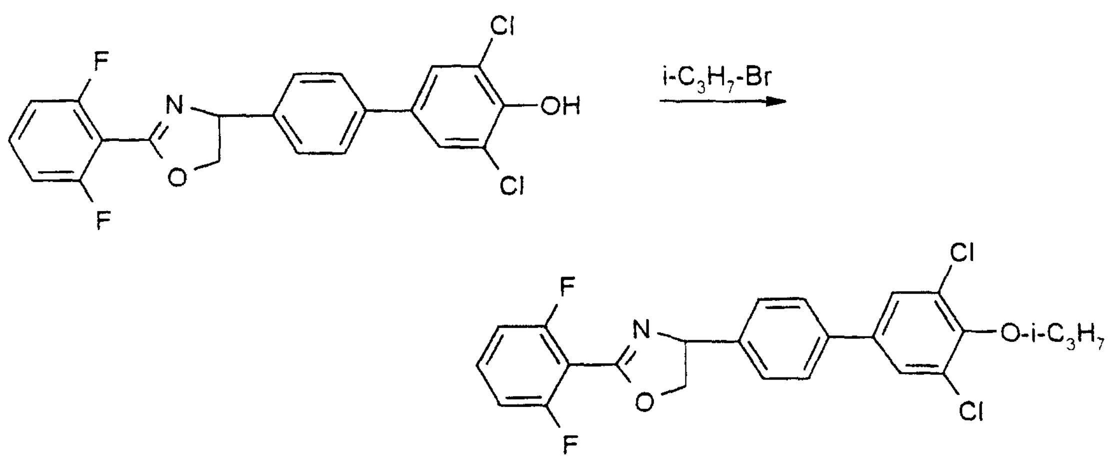


2-(2,6-Difluorphenyl)-4-(4-(4-(propyl-2-oxy)-3,5-dichlorphenyl)phenyl)-4,5-dihydro-oxazolin

2,9 g (7 mMol) 2-(2,6-Difluorphenyl)-4-(4-(4-hydroxy-3,5-dichlorphenyl)phenyl)-4,5-dihydro-oxazolin werden in 50 ml Acetonitril und 1,9 g (14 mMol) Pottasche und 8,6 g (70 mMol) 2-Propylbromid 6 Stunden unter Rückfluß erhitzt. Man gießt die Reaktionsmischung in Wasser und extrahiert das Produkt mit Methylenchlorid. Die org. Phase wird nach dem Waschen mit Wasser im Vakuum eingedampft und der Rückstand an Kieselgel im System Cyclohexan/Essigester (2:1) chromatographiert. Man erhält 1,6 g (2-(2,6-Difluorphenyl)-4-(4-(4-(propyl-2-oxy)-3,5-dichlorphenyl)phenyl)-4,5-dihydro-oxazolin als Öl von log p (pH 7,5) = 5,65 (Ausbeute: 49,5 % d.Th.).

Analog bzw. gemäß den allgemeinen Vorschriften zur Herstellung werden die in den folgenden Tabellen aufgeführten Verbindungen der Formel (I) erhalten:

**Tabelle 1**

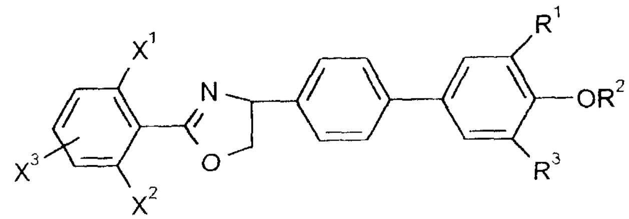 (I)

| Bsp.-Nr. | $X^1$ | $X^2$ | $X^3$ | $R^1$ | $R^2$ | $R^3$ | physik. Charakterisierung |
|---|---|---|---|---|---|---|---|
| I-2 | F | F | H | $-CH_2-CH_2-CH_3$ | $-CH(CH_3)_2$ | H | Fp. 60-62°C |
| I-3 | F | F | H | $CH_3$ | $-CH(CH_3)_2$ | H | Fp. 68-70°C |
| I-4 | F | F | H | $CH_3$ | 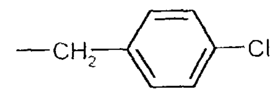 | H | Fp. 126-130°C |
| I-5 | F | F | H | $-CH_2-CH_2-CH_3$ | 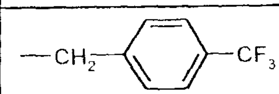 | H | Fp. 106-108°C |
| I-6 | F | F | H | $CH_3$ | 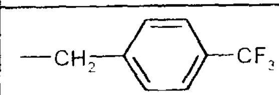 | H | Fp. 119-122°C |
| I-7 | F | F | H | $CH_3$ | $-CH(CH_3)C_2H_5$ | H | log p*: 5,60 (pH = 7,5) |

| Bsp.-Nr. | $X^1$ | $X^2$ | $X^3$ | $R^1$ | $R^2$ | $R^3$ | physik. Charakterisierung |
|---|---|---|---|---|---|---|---|
| I-8 | H | $CH_3$ | H | $-CH_2-CH_2-CH_3$ | 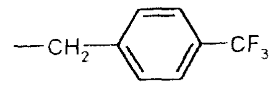 | H | log p: 7,07 (pH = 7,5) |
| I-9 | H | $CH_3$ | H | $-CH_2-CH_2-CH_3$ | $-CH(CH_3)C_2H_5$ | H | log p: 7,28 (pH = 7,5) |
| I-10 | F | F | H | $-CH_2-CH_2-CH_3$ | $-CH(CH_3)C_2H_5$ | H | log p: 6,33 (pH = 7,5) |
| I-11 | F | F | H | $-CH_2CH_2-CH_3$ | 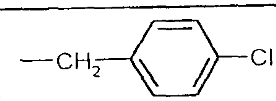 | H | Fp. 106-108°C |
| I-12 | H | Cl | H | $CH_3$ | $-CH(CH_3)_2$ | H | log p: 5,56 (pH = 7,5) |
| I-13 | H | Cl | H | $CH_3$ | 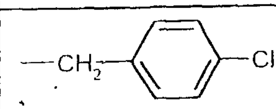 | H | log p: 6,15 (pH = 7,5) |
| I-14 | H | Br | H | $CH_3$ | $-CH(CH_3)_2$ | H | log p: 5,63 (pH = 7,5) |
| I-15 | H | Br | H | $CH_3$ | 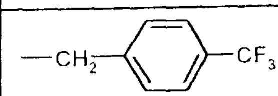 | H | Fp. 88-93°C |
| I-16 | H | Br | H | $CH_3$ | $-CH(CH_3)C_2H_5$ | H | log p: 6,12 (pH = 7,5) |

| Bsp.-Nr. | $X^1$ | $X^2$ | $X^3$ | $R^1$ | $R^2$ | $R^3$ | physik. Charakterisierung |
|---|---|---|---|---|---|---|---|
| I-17 | H | Cl | H | $CH_3$ | $-CH(CH_3)-CH(OH)CH_3$ | H | log p: 4,08/4,13 (pH = 7,5) |
| I-18 | H | Br | H | $CH_3$ | 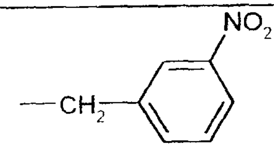 | H | Fp. 118-120°C |
| I-19 | F | F | H | Cl | $-CH(CH_3)_2$ | H | log p (pH 7,5): 4,96 |
| I-20 | F | F | H | Cl | 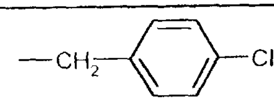 | H | Fp. 125-128°C |
| I-21 | F | F | H | Cl | 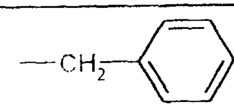 | H | Fp. 126°C |
| I-22 | F | F | H | Cl | 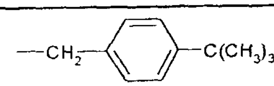 | H | Fp. 148°C |
| I-23 | F | F | H | Cl | 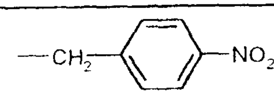 | H | Fp. 122°C |

| Bsp.-Nr. | $X^1$ | $X^2$ | $X^3$ | $R^1$ | $R^2$ | $R^3$ | physik. Charakterisierung |
|---|---|---|---|---|---|---|---|
| I-24 | F | F | H | Cl | $-CH_2-CH(CH_3)_2$ | H | Fp. 78-80°C |
| I-25 | F | F | H | Cl | $-(CH_2)_3CH_3$ | H | Fp. 94-96°C |
| I-26 | F | F | H | Cl | $-CH_2-CH=CH_2$ | H | Fp. 85°C |
| I-27 | F | F | H | Cl | $-CH_2-C(CH_3)=CH_2$ | H | Fp. 83-85°C |
| I-28 | F | F | H | Cl | $-CH_2CH_3$ | H | Fp. 130-132°C |
| I-29 | F | F | H | Cl | 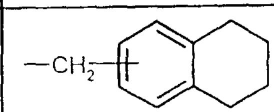 | H | Isomerengemisch log p 6,24 u. 6,32 (pH = 7,5) |
| I-30 | H | Cl | H | Cl | $-CH(CH_3)_2$ | H | log p (pH 7,5): 5,37 |
| I-31 | F | F | H | Cl | 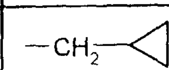 | H | Fp. 84-85°C |

| Bsp.-Nr. | $X^1$ | $X^2$ | $X^3$ | $R^1$ | $R^2$ | $R^3$ | physik. Charakterisierung |
|---|---|---|---|---|---|---|---|
| I-32 | H | F | H | Cl | $CH(CH_3)_2$ | H | log p (pH 7,5): 5,04 |
| I-33 | H | Cl | H | Cl | 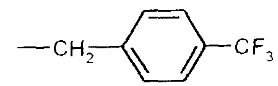 | H | Fp. 158-160°C |
| I-34 | H | Cl | H | Cl | $CH_2CH(CH_3)_2$ | H | log p (pH 7,5): 6,03 |
| I-35 | H | Cl | H | $CH_2$-$CH_2$-$CH_3$ | $CH(CH_3)CH(OH)CH_3)$ | H | log p: 4,79/4,86 (pH 7,5) |
| I-36 | H | Cl | H | $CH_2$-$CH_2$-$CH_3$ | $CH(CH_3)_2$ | H | log p: 6,35 (pH 7,5) |
| I-37 | H | Cl | H | $CH_2$-$CH_2$-$CH_3$ | $CH_2CH(CH_3)_2$ | H | log p: 6,95 (pH 7,5) |
| I-38 | H | Cl | H | $CH_2$-$CH_2$-$CH_3$ | 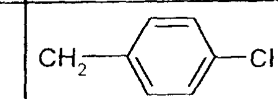 | H | log p: 6,79 (pH 7,5) |
| I-39 | H | Cl | H | $CH_2$-$CH_2$-$CH_3$ | $CH_2$-$CH(OH)CH_3$ | H | log p: 4,47 (pH 7,5) |

| Bsp.-Nr. | $X^1$ | $X^2$ | $X^3$ | $R^1$ | $R^2$ | $R^3$ | physik. Charakterisierung |
|---|---|---|---|---|---|---|---|
| I-40 | H | Br | H | $CH_2$-$CH_2$-$CH_3$ | $CH(CH_3)_2$ | H | log p: 6,40 (pH 7,5) |
| I-41 | H | I | H | $CH_3$ | 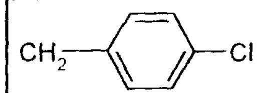 | H | Fp. 88-92°C |
| I-42 | H | Br | H | $CH_2$-$CH_2$-$CH_3$ | $CH_2C{\equiv}CH$ | H | Fp. 82-84°C |
| I-43 | H | Br | H | $CH_2$-$CH_2$-$CH_3$ | 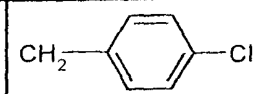 | H | log p: 6,84 (pH 7,5) |
| I-44 | F | F | H | $CH_3$ | $CH(CH_3)_2$ | $CH_3$ | log p: 5,23 (pH 7,5) |
| I-45 | F | F | H | $CH_2CH{=}CH_2$ | $CH(CH_3)_2$ | H | Fp. 86-87°C |
| I-46 | F | F | H | $CH_2CH{=}CH_2$ | 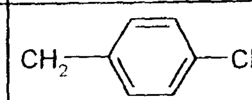 | H | Fp. 96-98°C |
| I-47 | F | F | H | $CH_3$ | $CH_2C(CH_3){=}CH_2$ | H | Fp. 106-108°C |
| I-48 | F | F | H | $CH_3$ | $CH_2$-$CH_2$-$CH_3$ | H | Fp. 112-114°C |

| Bsp.-Nr. | $X^1$ | $X^2$ | $X^3$ | $R^1$ | $R^2$ | $R^3$ | physik. Charakterisierung |
|---|---|---|---|---|---|---|---|
| I-49 | F | F | H | $CH_3$ | 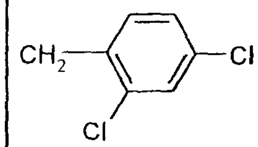 | H | Fp. 118-120°C |
| I-50 | H | Br | H | $CH_2$-$CH_2$-$CH_3$ | 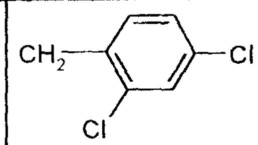 | H | Fp. 108-110°C |
| I-51 | H | Br | H | $CH_2$-$CH_2$-$CH_3$ | 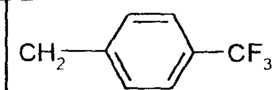 | H | Fp. 117-119°C |
| I-52 | H | $CH_3$ | H | $CH_2$-$CH_2$-$CH_3$ | $CH(CH_3)_2$ | H | log p: 6,78 (pH 7,5) |
| I-53 | F | F | H | $CH_2$-$CH_2$-$CH_3$ | 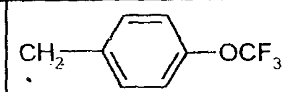 | H | Fp. 78-80°C |
| I-54 | F | F | H | $CH_2CH=CH_2$ | $CH_2CH=CH_2$ | H | Fp. 106-108°C |
| I-55 | H | $CH_3$ | H | $CH_3$ | $CH_2CH=CH_2$ | H | log p: 5,64 (pH 7,5) |
| I-56 | H | $CH_3$ | H | $CH_3$ | 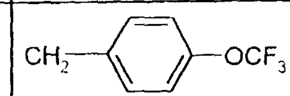 | H | log p: 6,50 (pH 7,5) |

| Bsp.-Nr. | $X^1$ | $X^2$ | $X^3$ | $R^1$ | $R^2$ | $R^3$ | physik. Charakterisierung |
|---|---|---|---|---|---|---|---|
| I-57 | H | I | H | $CH_2$-$CH_2$-$CH_3$ | $CH(CH_3)_2$ | H | log p: 6,59 (pH 7,5) |
| I-58 | H | I | H | $CH_2$-$CH_2$-$CH_3$ | 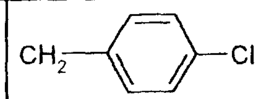 | H | log p: 7,03 (pH 7,5) |
| I-59 | F | F | H | $CH_2CH{=}CH_2$ | $CH(CH_3)C_2H_5$ | H | log p: 5,95 (pH 7,5) |
| I-60 | F | F | H | $CH_2$-$CH_2$-$CH_3$ | 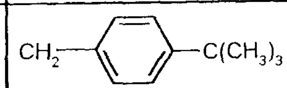 | H | log p: 7,14 (pH 7,5) |
| I-61 | H | Br | H | $CH_3$ | 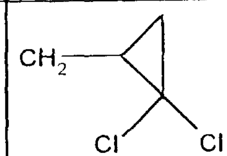 | H | log p: 5,76 (pH 7,5) |
| I-62 | H | I | H | $CH_2$-$CH_2$-$CH_3$ | $CH_2CH{=}CH_2$ | H | Fp. 73-75° |
| I-63 | F | F | H | $C_2H_5$ | $CH(CH_3)_2$ | H | log p: 5,54 (pH 7,5) |
| I-64 | H | $CH_3$ | H | $CH_2$-$CH_2$-$CH_3$ | $CH_2CH(CH_3)_2$ | H | log p: 4,48 (pH 7,5) |
| I-65 | F | F | H | $CH_3$ | 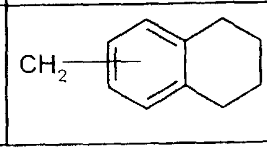 | H | log p: 6,44/6,55 (pH 7,5) |

| Bsp.-Nr. | $X^1$ | $X^2$ | $X^3$ | $R^1$ | $R^2$ | $R^3$ | physik. Charakterisierung |
|---|---|---|---|---|---|---|---|
| I-66 | F | F | H | $CH_2$-$CH_2$-$CH_3$ | $CH_2$-$CH_2$-$CH_3$ | H | Fp. 90-92°C |
| I-67 | H | I | H | $CH_3$ | $CH(CH_3)_2$ | H | log p: 5,81 (pH 7,5) |
| I-68 | F | F | H | $C_2H_5$ | 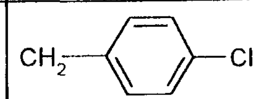 | H | Fp. 118-120°C |
| I-69 | H | Br | H | $CH_3$ | $CH(CH_3)CH(OH)CH_3$ | H | log p: 4,15/4,20 (pH 7,5) |
| I-70 | H | $CH_3$ | H | $CH_3$ | $CH(CH_3)_2$ | H | log p: 6,02 (pH 7,5) |
| I-71 | Cl | F | H | Cl | $CH(CH_3)_2$ | H | log p: 5,25 (pH 7,5) |
| I-72 | Cl | F | H | Cl | 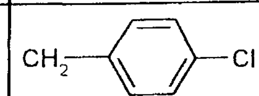 | H | Fp. 136°C |
| I-73 | Cl | F | H | Cl | $CH_2C{\equiv}CH$ | H | Fp. 138°C |
| I-74 | Cl | F | H | Cl | 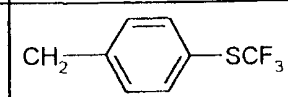 | H | Fp. 126°C |
| I-75 | Cl | F | H | Cl | $CH_2$-$CH_2$-$CH_3$ | H | Fp. 120°C |

| Bsp.-Nr. | $X^1$ | $X^2$ | $X^3$ | $R^1$ | $R^2$ | $R^3$ | physik. Charakterisierung |
|---|---|---|---|---|---|---|---|
| I-76 | Cl | F | H | Cl | $CH(CH_3)C_2H_5$ | H | log p: 5,69 (pH 7,5) |
| I-77 | Cl | F | H | Cl | $CH_2CH(CH_3)_2$ | H | log p: 5,89 (pH 7,5) |
| I-78 | Cl | F | H | Cl | 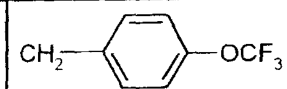 | H | log p: 5,94 (pH 7,5) |
| I-79 | Cl | F | H | Cl | 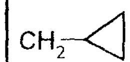 | H | Fp. 116°C |
| I-80 | Cl | F | H | Cl | $CH_2CH{=}CH_2$ | H | log p: 4,99 (pH 7,5) |
| I-81 | Cl | F | H | Cl | 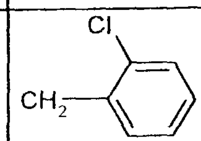 | H | log p: 5,94 (pH 7,5) |
| I-82 | Cl | F | H | $CH_2$-$CH_2$-$CH_3$ | 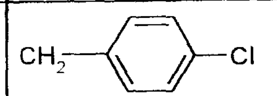 | H | Fp. 92°C |
| I-83 | Cl | F | H | Cl | n-$C_6H_{13}$ | H | log p: 6,68 (pH 7,5) |
| I-84 | Cl | F | H | $CH_2$-$CH_2$-$CH_3$ | $CH_2$-$CH_2$-$CH_3$ | H | Fp. 80°C |

| Bsp.-Nr. | $X^1$ | $X^2$ | $X^3$ | $R^1$ | $R^2$ | $R^3$ | physik. Charakterisierung |
|---|---|---|---|---|---|---|---|
| I-85 | Cl | F | H | $CH_2$-$CH_2$-$CH_3$ | $CH(CH_3)_2$ | H | log p: 6,20 (pH 7,5) |
| I-86 | Cl | F | H | $CH_2$-$CH_2$-$CH_3$ | $CH(CH_3)C_2H_5$ | H | log p: 6,62 (pH 7,5) |
| I-87 | H | F | H | Cl | $CH_2$-$CH_2$-$CH_3$ | H | log p: 5,22 (pH 7,5) |
| I-88 | H | F | H | Cl | 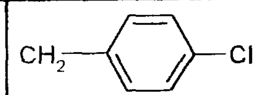 | H | Fp. 120°C |
| I-89 | H | F | H | Cl | $CH(CH_3)C_2H_5$ | H | log p: 5,49 (pH 7,5) |
| I-90 | H | F | H | Cl | $CH_2CH(CH_3)_2$ | H | log p: 5,70 (pH 7,5) |
| I-91 | Cl | F | H | $CH_2$-$CH_2$-$CH_3$ | $CH_2C{\equiv}CH$ | H | Fp. 82°C |
| I-92 | Cl | F | H | $CH_2$-$CH_2$-$CH_3$ | 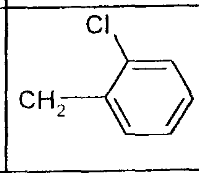 | H | log p: 6,78 (pH 7,5) |

| Bsp.-Nr. | $X^1$ | $X^2$ | $X^3$ | $R^1$ | $R^2$ | $R^3$ | physik. Charakterisierung |
|---|---|---|---|---|---|---|---|
| I-93 | Cl | F | H | Cl | 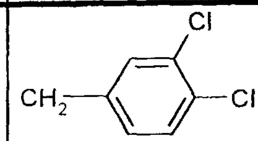 | H | Fp. 140°C |
| I-94 | Cl | F | H | $CH_2$-$CH_2$-$CH_3$ | 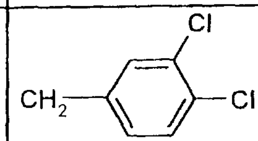 | H | Fp. 128°C |
| I-95 | H | F | H | Cl | 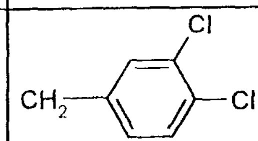 | H | Fp. 154°C |
| I-96 | H | F | H | Cl | n-$C_6H_{13}$ | H | log p: 6,48 (pH 7,5) |
| I-97 | H | F | H | Cl | $CH_2CH$=$CH_2$ | H | Fp. 92°C |
| I-98 | H | F | H | Cl | 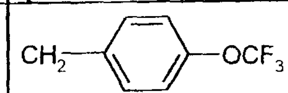 | H | Fp. 138°C |
| I-99 | Cl | F | H | Cl | 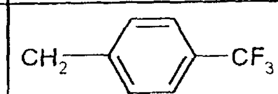 | H | Fp. 144°C |
| I-100 | Cl | F | H | $CH_2$-$CH_2$-$CH_3$ | 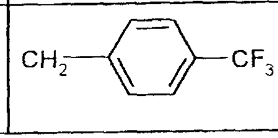 | H | Fp. 148°C |

| Bsp.-Nr. | $X^1$ | $X^2$ | $X^3$ | $R^1$ | $R^2$ | $R^3$ | physik. Charakterisierung |
|---|---|---|---|---|---|---|---|
| I-101 | Cl | F | H | $CH_2-CH_2-CH_3$ | 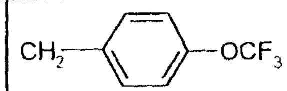 | H | Fp. 130°C |
| I-102 | Cl | F | H | $CH_2-CH_2-CH_3$ | 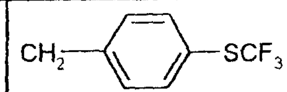 | H | Fp. 94°C |
| I-103 | Cl | F | H | $CH_2-CH_2-CH_3$ | $n-C_6H_{13}$ | H | Fp. 62°C |
| I-104 | Cl | F | H | $CH_2-CH_2-CH_3$ | $CH_2CH(CH_3)_2$ | H | log p: 6,82 (pH 7,5) |
| I-105 | Cl | F | H | $CH_2-CH_2-CH_3$ | $CH_2CH=CH_2$ | H | Fp. 48°C |
| I-106 | Cl | F | H | $CH_3$ | 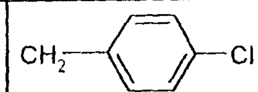 | H | Fp. 131°C |
| I-107 | Cl | F | H | $CH_3$ | 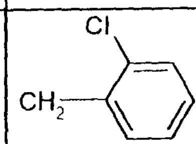 | H | Fp. 120°C |
| I-108 | H | F | H | $CH_3$ | 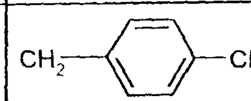 | H | Fp. 96°C |
| I-109 | H | F | H | $CH_3$ | 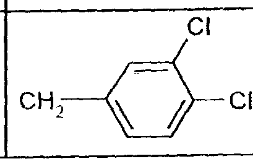 | H | log p: 6,25 (pH 7,5) |

Tabelle 2

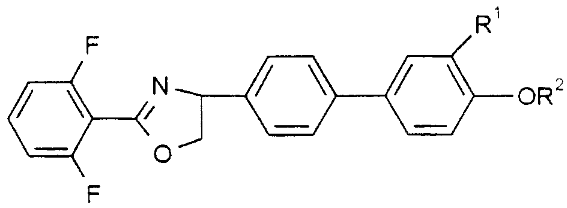

| Bsp.-Nr. | $R^1$ | $R^2$ | physik. Charakterisierung |
|---|---|---|---|
| I-110 | $CH_3$ | n-$C_6H_{13}$ | Fp. 95-97°C |
| I-111 | $CH_3$ | $CH_2CH{=}CH_2$ | Fp. 82-84°C |
| I-112 | $CH_3$ | $CH_2C{\equiv}CH$ | Fp. 116-18°C |
| I-113 | $CH_3$ | 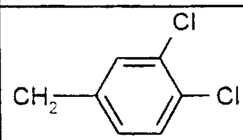 | Fp. 109-11°C |
| I-114 | $CH_3$ | 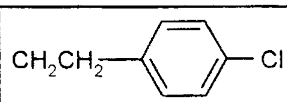 | log p: 5,90 (pH 7,5) |
| I-115 | n-$C_3H_7$ | $CH_2CH{=}CH_2$ | |
| I-116 | n-$C_3H_7$ | $CH_2C{\equiv}CH$ | Fp. 119-21°C |
| I-117 | n-$C_3H_7$ | 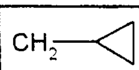 | |
| I-118 | n-$C_3H_7$ | 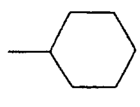 | |
| I-119 | n-$C_3H_7$ | 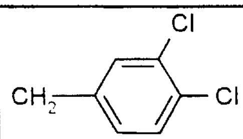 | Fp. 95-96°C |
| I-120 | n-$C_3H_7$ | 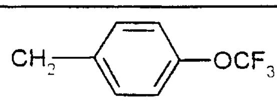 | Fp. 78-80°C |

| Bsp.-Nr. | $R^1$ | $R^2$ | physik. Charakterisierung |
|---|---|---|---|
| I-121 | n-$C_3H_7$ | 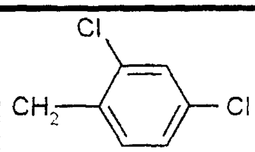 | |
| I-122 | n-$C_3H_7$ | 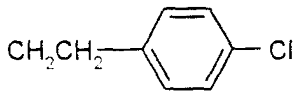 | Fp. 72-74°C |
| I-123 | $CH_2CH=CH_2$ | $CH_2C\equiv CH$ | Fp. 82-85°C |
| I-124 | $CH_2CH=CH_2$ | 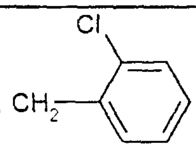 | |
| I-125 | $CH_2CH=CH_2$ | 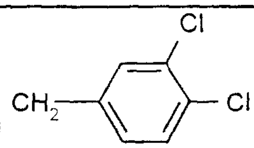 | Fp. 106-108°C |
| I-126 | $CH_2CH=CH_2$ | 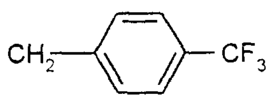 | Fp. 75-77°C |
| I-127 | $CH_2CH=CH_2$ | 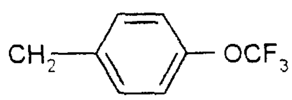 | Fp. 76-78°C |
| I-128 | $CH_2CH=CH_2$ | 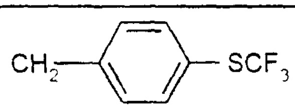 | Fp. 75-77°C |
| I-129 | $CH_2CH=CH_2$ | 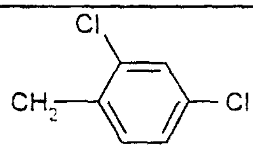 | log p: 6,73 (pH 7,5) |
| I-130 | $CH_2CH=CH_2$ | 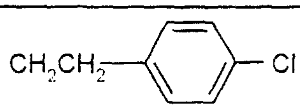 | Fp. 83-85°C |

**Tabelle 3**

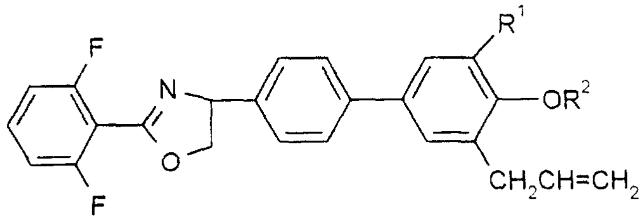

| Bsp.-Nr. | $R^1$ | $R^2$ | physik. Charakterisierung |
|---|---|---|---|
| I-131 | $CH_3$ | $i\text{-}C_3H_7$ | |
| I-132 | $CH_3$ | $s\text{-}C_4H_9$ | |
| I-133 | $CH_3$ | $CH_2CH{=}CH_2$ | |
| I-134 | $CH_3$ | 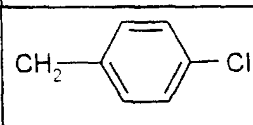 | Fp. 75°C |
| I-135 | $CH_3$ | 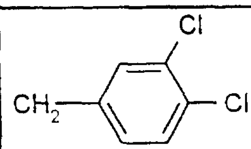 | |
| I-136 | $CH_3$ | 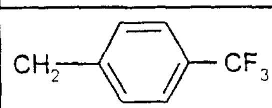 | Fp. 76°C |
| I-137 | $CH_3$ | 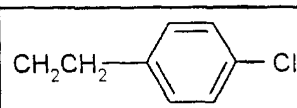 | |
| I-138 | $CH_2CH{=}CH_2$ | $i\text{-}C_3H_7$ | Fp. 78-80°C |
| I-139 | $CH_2CH{=}CH_2$ | $s\text{-}C_4H_9$ | log p: 6,39 (pH 7,5) |
| I-140 | $CH_2CH{=}CH_2$ | $CH_2CH{=}CH_2$ | Fp. 75-77°C |
| I-141 | $CH_2CH{=}CH_2$ | 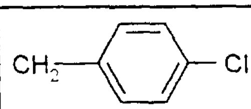 | Fp. 75-76°C |

| Bsp.-Nr. | $R^1$ | $R^2$ | physik. Charakterisierung |
|---|---|---|---|
| I-142 | $CH_2CH{=}CH_2$ | 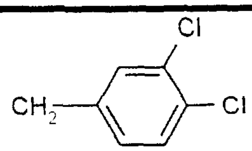 | log p: 6,99 (pH 7,5) |
| I-143 | $CH_2CH{=}CH_2$ | 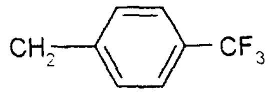 | Fp. 73-75°C |
| I-144 | $CH_2CH{=}CH_2$ | 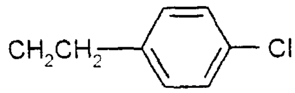 | |

**Tabelle 4**

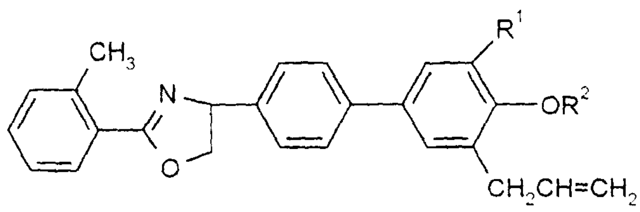

| Bsp.-Nr. | $R^1$ | $R^2$ | physik. Charakterisierung |
|---|---|---|---|
| I-145 | Cl | n-$C_6H_{13}$ | |
| I-146 | Cl | i-$C_3H_7$ | log p: 6,75 (pH 7,5) |
| I-147 | Cl | $CH_2C{\equiv}CH$ | |
| I-148 | Cl | 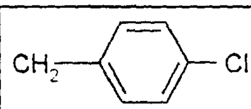 | log p: 7,32 (pH 7,5) |
| I-149 | Cl | 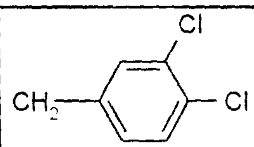 | log p: 7,36 (pH 7,5) |
| I-150 | Cl | 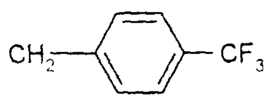 | log p: 7,16 (pH 7,5) |

**Tabelle 5**

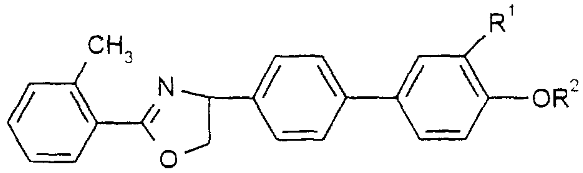

| Bsp.-Nr. | $R^1$ | $R^2$ | physik. Charakterisierung |
|---|---|---|---|
| I-151 | $CH_3$ | $CH_2C{\equiv}CH$ | |
| I-152 | $CH_3$ | 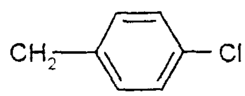 | log p: 6,51 (pH 7,5) |
| I-153 | $CH_3$ | 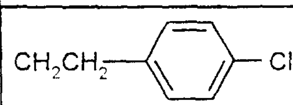 | |
| I-154 | Cl | $i\text{-}C_3H_7$ | Fp.108-10°C |
| I-155 | Cl | $CH_2CH{\equiv}CH_2$ | log p:5,47 (pH 7,5) |
| I-156 | Cl | $CH_2C{\equiv}CH$ | log p: 4,81 (pH 7,5) |
| I-157 | Cl | 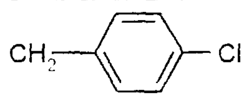 | Fp. 125-27°C |
| I-158 | Cl | 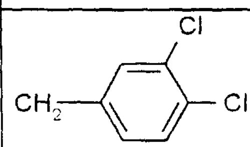 | Fp. 110-12°C |
| I-159 | Cl | 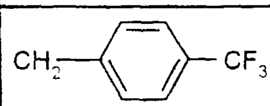 | Fp. 125-28°C |
| I-160 | Cl | 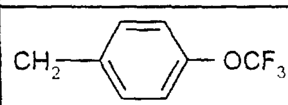 | Fp. 97-99°C |
| I-161 | Cl | 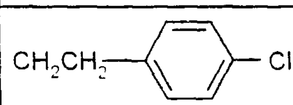 | |

Tabelle 6

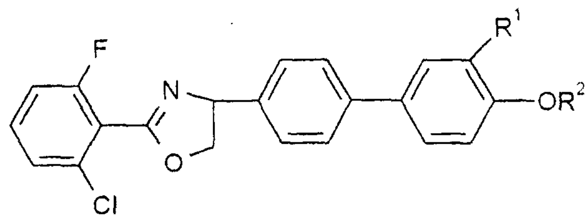

| Bsp.-Nr. | $R^1$ | $R^2$ | physik. Charakterisierung |
|---|---|---|---|
| I-162 | $CH_3$ | $n\text{-}C_3H_7$ | Fp. 98°C |
| I-163 | $CH_3$ | $i\text{-}C_3H_7$ | log p: 5,45 (pH 7,5) |
| I-164 | $CH_3$ | $i\text{-}C_4H_9$ | Fp. 91°C |
| I-165 | $CH_3$ | $s\text{-}C_4H_9$ | log p: 5,93 (pH 7,5) |
| I-166 | $CH_3$ | $n\text{-}C_6H_{13}$ | Fp. 68°C |
| I-167 | $CH_3$ | $CH_2CH=CH_2$ | Fp. 77°C |
| I-168 | $CH_3$ | $CH_2C\equiv CH$ | Fp. 149°C |
| I-169 | $CH_3$ | 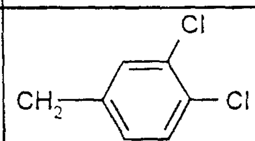 | Fp. 148°C |
| I-170 | $CH_3$ | 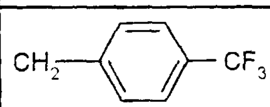 | Fp. 148°C |
| I-171 | $CH_3$ | 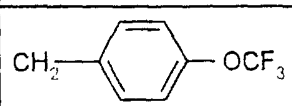 | Fp. 113°C |
| I-172 | $CH_3$ | 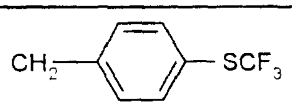 | log p: 6,37 (pH 7,5) |
| I-173 | $CH_3$ | 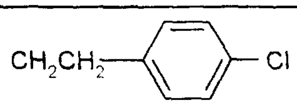 | log p: 6,21 (pH 7,5) |

| Bsp.-Nr. | $R^1$ | $R^2$ | physik. Charakterisierung |
|---|---|---|---|
| I-174 | $n\text{-}C_3H_7$ | 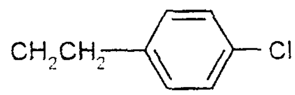 | log p: 6,82 (pH 7,5) |
| I-175 | Cl | 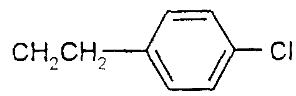 | |
| I-176 | $CH_2CH=CH_2$ | $n\text{-}C_3H_7$ | log p: 5,98 (pH 7,5) |
| I-177 | $CH_2CH=CH_2$ | $i\text{-} C_3H_7$ | log p: 5,82 (pH 7,5) |
| I-178 | $CH_2CH=CH_2$ | $i\text{-}C_4H_9$ | log p: 6,47 (pH 7,5) |
| I-179 | $CH_2CH=CH_2$ | $s\text{-} C_4H_9$ | log p: 6,33 (pH 7,5) |
| I-180 | $CH_2CH=CH_2$ | $n\text{-}C_6H_{13}$ | Fp. 48°C |
| I-181 | $CH_2CH=CH_2$ | $CH_2CH=CH_2$ | log p: 5,49 (pH 7,5) |
| I-182 | $CH_2CH=CH_2$ | $CH_2C\equiv CH$ | log p: 4,81 (pH 7,5) |
| I-183 | $CH_2CH=CH_2$ | 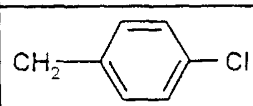 | log p: 6,30 (pH 7,5) |
| I-184 | $CH_2CH=CH_2$ | 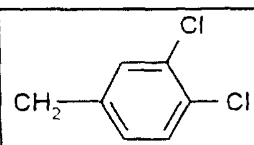 | log p: 6,73 (pH 7,5) |
| I-185 | $CH_2CH=CH_2$ | 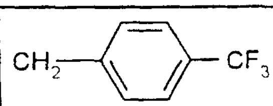 | Fp. 73°C |
| I-186 | $CH_2CH=CH_2$ | 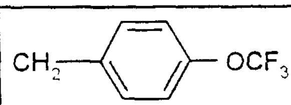 | log p: 6,27 (pH 7,5) |

| Bsp.-Nr. | $R^1$ | $R^2$ | physik. Charakterisierung |
|---|---|---|---|
| I-187 | $CH_2CH=CH_2$ | 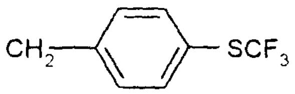 | log p: 6,56 (pH 7,5) |
| I-188 | $CH_2CH=CH_2$ | 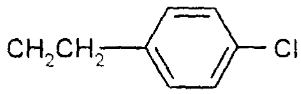 | log p: 6,45 (pH 7,5) |

Tabelle 7

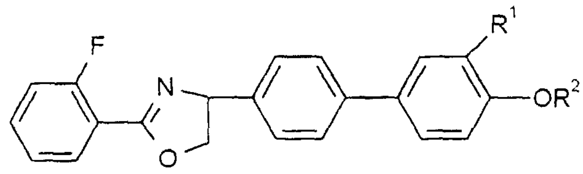

| Bsp.-Nr. | $R^1$ | $R^2$ | physik. Charakterisierung |
|---|---|---|---|
| I-189 | $CH_3$ | $n\text{-}C_3H_7$ | Fp. 79°C |
| I-190 | $CH_3$ | $i\text{-}C_3H_7$ | log p: 5,23 (pH 7,5) |
| I-191 | $CH_3$ | $i\text{-}C_4H_9$ | log p: 5,89 (pH 7,5) |
| I-192 | $CH_3$ | $s\text{-} C_4H_9$ | log p: 5,70 (pH 7,5) |
| I-193 | $CH_3$ | $n\text{-}C_6H_{13}$ | Fp. 68°C |
| I-194 | $CH_3$ | $CH_2CH{=}CH_2$ | Fp. 71°C |
| I-195 | $CH_3$ | $CH_2C{\equiv}CH$ | Fp. 99°C |
| I-196 | $CH_3$ | $CH(CH_3)C{\equiv}CH$ | log p: 4,68 (pH 7,5) |
| I-197 | $CH_3$ | 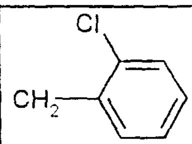 | log p: 5,91 (pH 7,5) |
| I-198 | $CH_3$ | 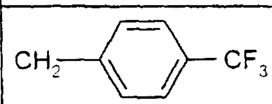 | Fp. 109°C |
| I-199 | $CH_3$ | 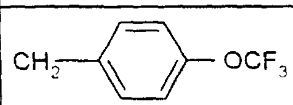 | log p: 5,90 (pH 7,5) |
| I-200 | $CH_3$ | 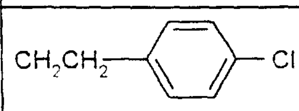 | log p: 5,98 (pH 7,5) |
| I-201 | $n\text{-}C_3H_7$ | $n\text{-}C_3H_7$ | Fp. 73°C |

| Bsp.-Nr. | $R^1$ | $R^2$ | physik. Charakterisierung |
|---|---|---|---|
| I-202 | n-$C_3H_7$ | i-$C_3H_7$ | log p: 6,03 (pH 7,5) |
| I-203 | n-$C_3H_7$ | n-$C_4H_9$ | Fp. 79°C |
| I-204 | n-$C_3H_7$ | i- $C_4H_9$ | log p: 6,61 (pH 7,5) |
| I-205 | n-$C_3H_7$ | s- $C_4H_9$ | log p: 6,47 |
| I-206 | n-$C_3H_7$ | n-$C_6H_{13}$ | Fp. 74°C |
| I-207 | n-$C_3H_7$ | $CH_2CH{=}CH_2$ | Fp. 72°C |
| I-208 | n-$C_3H_7$ | $CH_2C{\equiv}CH$ | Fp. 100°C |
| I-209 | n-$C_3H_7$ | $CH(CH_3)C{\equiv}CH$ | log p: 5,32 (pH 7,5) |
| I-210 | n-$C_3H_7$ | 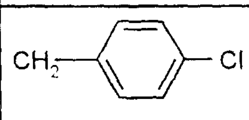 | log p: 6,45 (pH 7,5) |
| I-211 | n-$C_3H_7$ | 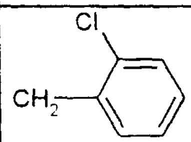 | log p: 6,56 (pH 7,5) |
| I-212 | n-$C_3H_7$ | 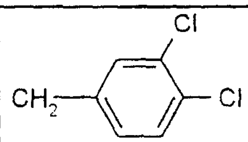 | log p: 6,93 (pH 7,5) |
| I-213 | n-$C_3H_7$ | 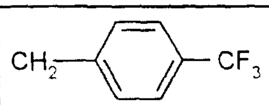 | log p: 6,35 (pH 7,5) |
| I-214 | n-$C_3H_7$ | 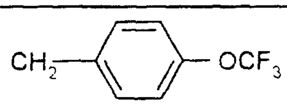 | log p: 6,44 (pH 7,5) |
| I-215 | n-$C_3H_7$ | 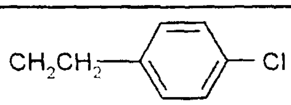 | log p: 6,57 (pH 7,5) |
| I-216 | Cl | n-$C_3H_7$ | log p: 5,22 (pH 7,5) |
| I-217 | Cl | i-$C_4H_9$ | log p: 5,70 (pH 7,5) |

| Bsp.-Nr. | $R^1$ | $R^2$ | physik. Charakterisierung |
|---|---|---|---|
| I-218 | Cl | s-$C_4H_9$ | log p: 5,49 (pH 7,5) |
| I-219 | Cl | n-$C_6H_{13}$ | log p: 6,48 (pH 7,5) |
| I-220 | Cl | $CH_2CH{=}CH_2$ | Fp. 92°C |
| I-221 | Cl | $CH_2C{\equiv}CH$ | log p: 4,16 (pH 7,5) |
| I-222 | Cl | $CH(CH_3)C{\equiv}CH$ | log p: 4,58 (pH 7,5) |
| I-223 | Cl | 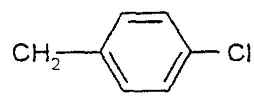 | Fp. 120°C |
| I-224 | Cl | 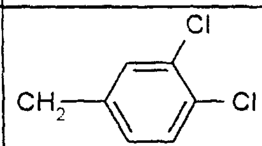 | Fp. 154°C |
| I-225 | Cl | 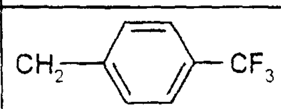 | Fp. 140°C |
| I-226 | Cl | 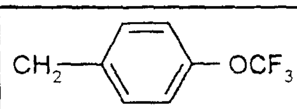 | Fp. 138°C |
| I-227 | Cl | 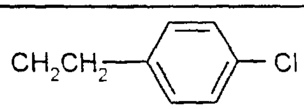 | log p: 5,91 (pH 7,5) |

**Tabelle 8**

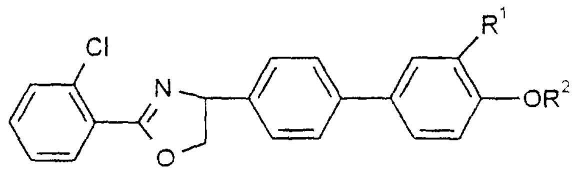

| Bsp.-Nr. | $R^1$ | $R^2$ | physik. Charakterisierung |
|---|---|---|---|
| I-228 | $CH_3$ | n-$C_3H_7$ | |
| I-229 | $CH_3$ | i-$C_3H_7$ | log p: 5,56 (pH 7,5) |
| I-230 | $CH_3$ | i-$C_4H_9$ | |
| I-231 | $CH_3$ | n-$C_6H_{13}$ | Fp. 68-70°C |
| I-232 | $CH_3$ | $CH_2CH{=}CH_2$ | Fp. 85-87°C |
| I-233 | $CH_3$ | $CH_2C{\equiv}CH$ | Fp. 96-98°C |
| I-234 | $CH_3$ | 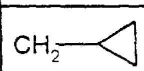 | log p: 5,57 (pH 7,5) |
| I-235 | $CH_3$ | 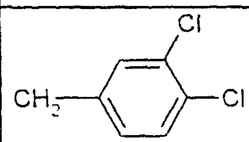 | log p: 6,56 (pH 7,5) |
| I-236 | $CH_3$ | 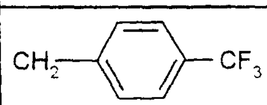 | log p: 6,06 (pH 7,5) |
| I-237 | $CH_3$ | 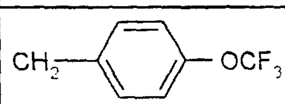 | Fp. 83-84°C |
| I-238 | $CH_3$ | 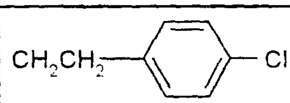 | log p: 6,31 (pH 7,5) |
| I-239 | n-$C_3H_7$ | n-$C_3H_7$ | |
| I-240 | n-$C_3H_7$ | i-$C_3H_7$ | log p: 6,35 (pH 7,5) |
| I-241 | n-$C_3H_7$ | n-$C_6H_{13}$ | |

| Bsp.-Nr. | $R^1$ | $R^2$ | physik. Charakterisierung |
|---|---|---|---|
| I-242 | n-$C_3H_7$ | $CH_2CH{=}CH_2$ | |
| I-243 | n-$C_3H_7$ | $CH_2C{\equiv}CH$ | |
| I-244 | n-$C_3H_7$ | 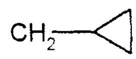 | |
| I-245 | n-$C_3H_7$ | 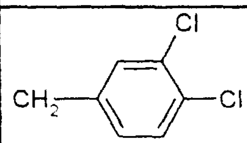 | |
| I-246 | n-$C_3H_7$ | 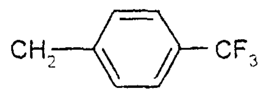 | |
| I-247 | n-$C_3H_7$ | 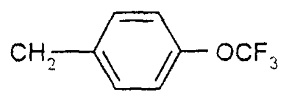 | |
| I-248 | n-$C_3H_7$ | 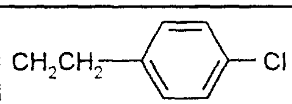 | |
| I-249 | $CH_2CH{=}CH_2$ | n-$C_3H_7$ | Fp. 56-58°C |
| I-250 | $CH_2CH{=}CH_2$ | i-$C_3H_7$ | log p: 5,93 (pH 7,5) |
| I-251 | $CH_2CH{=}CH_2$ | i-$C_4H_9$ | log p: 6,52 (pH 7,5) |
| I-252 | $CH_2CH{=}CH_2$ | $CH_2CH{=}CH_2$ | Fp. 58-60°C |
| I-253 | $CH_2CH{=}CH_2$ | $CH_2C{\equiv}CH$ | Fp. 74-76°C |
| I-254 | $CH_2CH{=}CH_2$ | 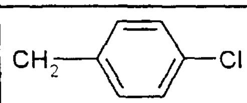 | log p: 6,39 (pH 7,5) |
| I-255 | $CH_2CH{=}CH_2$ | 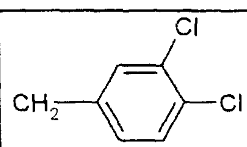 | log p: 6,83 (pH 7,5) |
| I-256 | $CH_2CH{=}CH_2$ | 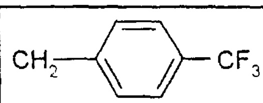 | log p: 6,30 (pH 7,5) |

| Bsp.-Nr. | $R^1$ | $R^2$ | physik. Charakterisierung |
|---|---|---|---|
| I-257 | $CH_2CH=CH_2$ | 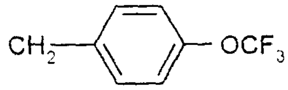 | log p: 6,83 (pH 7,5) |
| I-258 | $CH_2CH=CH_2$ | 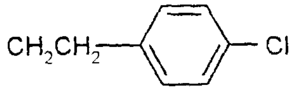 | |

**Tabelle 9**

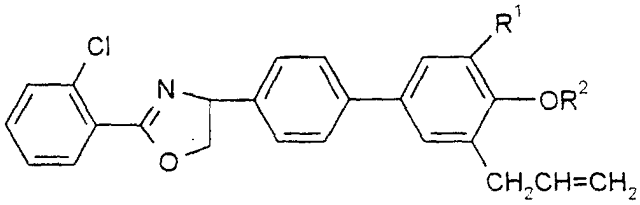

| Bsp.-Nr. | $R^1$ | $R^2$ | physik. Charakterisierung |
|---|---|---|---|
| I-259 | | | |
| I-260 | $CH_3$ | i-$C_3H_7$ | |
| I-261 | $CH_3$ | n-$C_4H_9$ | |
| I-262 | $CH_3$ | i- $C_4H_9$ | |
| I-263 | $CH_3$ | $CH_2CH{=}CH_2$ | |
| I-264 | $CH_3$ | $CH_2C{\equiv}CH$ | |
| I-265 | $CH_3$ | 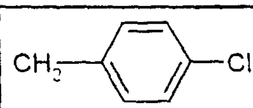 | log p: 6,64 (pH 7,5) |
| I-266 | $CH_3$ | 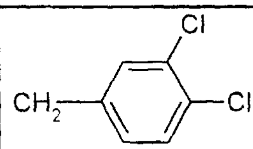 | |
| I-267 | $CH_3$ | 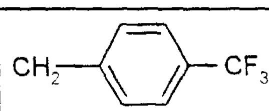 | |
| I-268 | $CH_3$ | 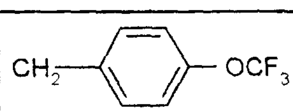 | |
| I-269 | $CH_3$ | 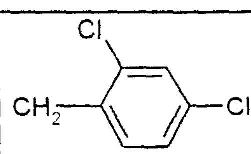 | |

**Tabelle 10**

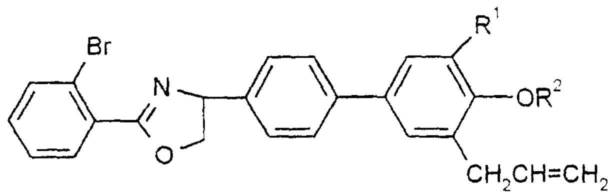

| Bsp.-Nr. | $R^1$ | $R^2$ | physik. Charakte-risierung |
|---|---|---|---|
| I-270 | $CH_2CH=CH_2$ | n-$C_3H_7$ | |
| I-271 | $CH_2CH=CH_2$ | i-$C_3H_7$ | |
| I-272 | $CH_2CH=CH_2$ | n-$C_6H_{13}$ | |
| I-273 | $CH_2CH=CH_2$ | $CH_2CH=CH_2$ | |
| I-274 | $CH_2CH=CH_2$ | $CH_2C≡CH$ | |
| I-275 | $CH_2CH=CH_2$ | 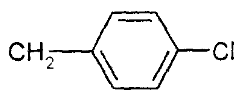 | |
| I-276 | $CH_2CH=CH_2$ | 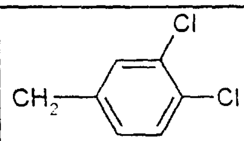 | |
| I-277 | $CH_2CH=CH_2$ | 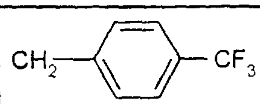 | |
| I-278 | $CH_2CH=CH_2$ | 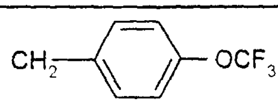 | |
| I-279 | $CH_2CH=CH_2$ | 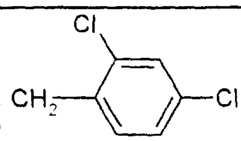 | |
| I-280 | Cl | n-$C_3H_7$ | |
| I-281 | Cl | i-$C_3H_7$ | log p: 6,35 (pH 7,5) |
| I-282 | Cl | n-$C_6H_{13}$ | log p: 7,36 (pH 7,5) |

| Bsp.-Nr. | $R^1$ | $R^2$ | physik. Charakterisierung |
|---|---|---|---|
| I-283 | Cl | $CH_2CH=CH_2$ | log p: 6,05 (pH 7,5) |
| I-284 | Cl | $CH_2C{\equiv}CH$ | log p: 5,37 (pH 7,5) |
| I-285 | Cl | 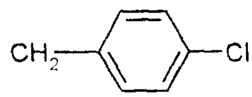 | log p: 6,89 (pH 7,5) |
| I-286 | Cl | 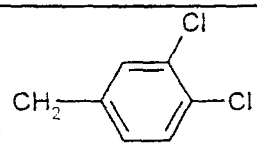 | log p: 7,36 (pH 7,5) |
| I-287 | Cl | 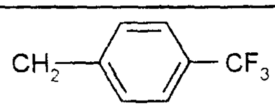 | log p: 6,79 (pH 7,5) |
| I-288 | Cl | 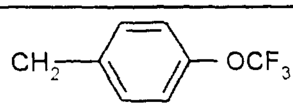 | log p: 6,88 (pH 7,5) |
| I-289 | Cl | 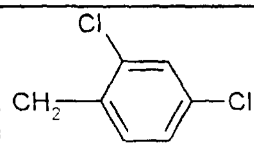 | log p: 7,36 (pH 7,5) |

## Tabelle 11

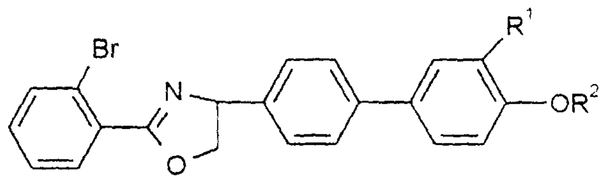

| Bsp.-Nr. | $R^1$ | $R^2$ | physik. Charakte-risierung |
|---|---|---|---|
| I-290 | $CH_2CH{=}CH_2$ | $n\text{-}C_3H_7$ | |
| I-291 | $CH_2CH{=}CH_2$ | $i\text{-}C_3H_7$ | log p: 6,02 (pH 7,5) |
| I-292 | $CH_2CH{=}CH_2$ | $n\text{-}C_6H_{13}$ | |
| I-293 | $CH_2CH{=}CH_2$ | $CH_2CH{=}CH_2$ | log p: 5,66 (pH 7,5) |
| I-294 | $CH_2CH{=}CH_2$ | $CH_2C{\equiv}CH$ | |
| I-295 | $CH_2CH{=}CH_2$ | 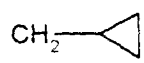 | log p: 5,99 (pH 7,5) |
| I-296 | $CH_2CH{=}CH_2$ | 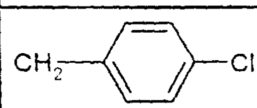 | log p: 6,44 (pH 7,5) |
| I-297 | $CH_2CH{=}CH_2$ | 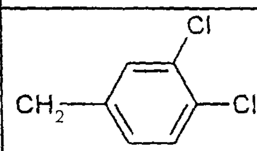 | |
| I-298 | $CH_2CH{=}CH_2$ | 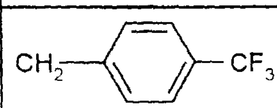 | Fp. 105°C |
| I-299 | $CH_2CH{=}CH_2$ | 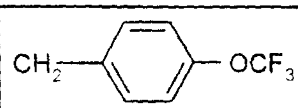 | |

| Bsp.-Nr. | $R^1$ | $R^2$ | physik. Charakterisierung |
|---|---|---|---|
| I-300 | $CH_2CH=CH_2$ | 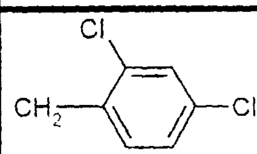 | |
| I-301 | Cl | $n-C_3H_7$ | Fp. 78-80°C |
| I-302 | Cl | $i-C_3H_7$ | log p: 5,42 (pH 7,5) |
| I-303 | Cl | $n-C_4H_9$ | |
| I-304 | Cl | $CH_2CH=CH_2$ | log p: 5,13 (pH 7,5) |
| I-305 | Cl | $CH_2C\equiv CH$ | Fp. 112-114°C |
| I-306 | Cl | 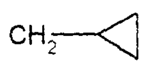 | |
| I-307 | Cl | 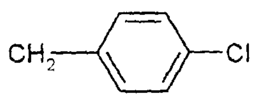 | Fp. 108-110°C |
| I-308 | Cl | 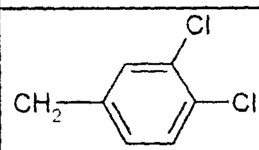 | Fp. 130-32°C |
| I-309 | Cl | 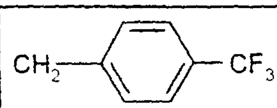 | Fp. 160-162°C |
| I-310 | Cl | 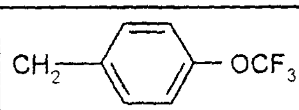 | Fp. 125-127°C |
| I-311 | Cl | 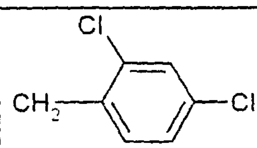 | |

**Tabelle 12**

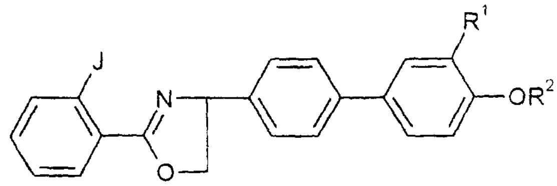

| Bsp.-Nr. | $R^1$ | $R^2$ | physik. Charakte-risierung |
|---|---|---|---|
| I-312 | $CH_2CH_2CH_3$ | $CH_2C{\equiv}CH$ | Fp. 75-79°C |
| I-313 | $CH_2CH_2CH_3$ | 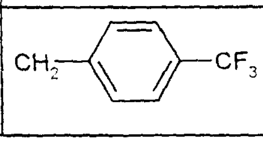 | Fp. 113-115°C |

I-314 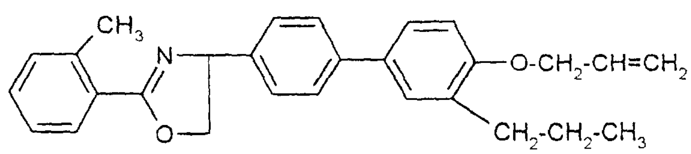 log p: 6,44 (pH 7,5)

*) log p: Dekadischer Logarithmus des n-Octanol/Wasser-Verteilungkoeffizienten, bestimmt durch HPLC-Analytik an reversed phase mit $H_2O/CH_3CN$

**Tabelle 13**

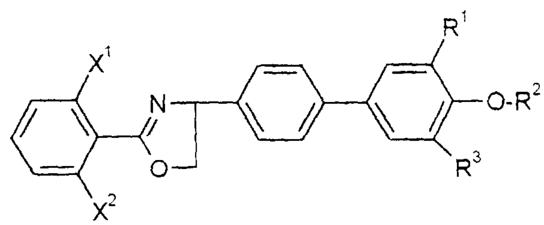

| Bsp.-Nr. | $X^1$ | $X^2$ | $R^1$ | $R^3$ | $R^2$ | physik. Charakter |
|---|---|---|---|---|---|---|
| I-315 | H | Cl | $CH_3$ | H | 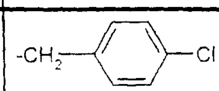 | log p: 6.15 (pH = 7,5) |
| I-316 | H | Br | $CH_3$ | H | $-C_3H_7-i$ | log p: 5.63 (pH = 7,5) |
| I-317 | H | Cl | $CH_3$ | H | $-CH(CH_3)-CH(OH)CH_3$ | log p: 4.08/4.13 (pH ) 7,5) |

| Bsp.-Nr. | $X^1$ | $X^2$ | $R^1$ | $R^3$ | $R^2$ | physik. Charakter |
|---|---|---|---|---|---|---|
| I-318 | F | F | $C_2H_5$ | H | 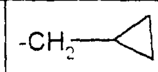 | Fp. 74-75°C |
| I-319 | F | F | $C_2H_5$ | H | $-CH_2-CH=CH_2$ | Fp. 86-88°C |
| I-320 | F | F | $C_2H_5$ | H | $-CH_2-C\equiv CH$ | Fp. 90-92°C |
| I-321 | F | F | $C_2H_5$ | H | $-CH(CH_3)C_2H_5$ | log p: 5.97 (pH = 7,5) |
| I-322 | F | F | $C_2H_5$ | H | $-CH_2-CH(CH_3)_2$ | Fp. 70-72°C |
| I-323 | H | Cl | $C_2H_5$ | H | $-CH_2-CH=CH_2$ | Fp. 72-74°C |
| I-324 | F | F | $CH_3$ | H | 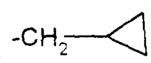 | Fp. 70-72°C |
| I-325 | F | F | $C_2H_5$ | H | 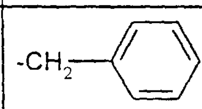 | Fp.112-15°C |
| I-326 | H | Cl | $C_2H_5$ | H | $-CH_2-C\equiv CH$ | Fp. 78-80°C |
| I-327 | F | F | $C_2H_5$ | H | 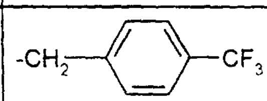 | Fp.112-15°C |
| I-328 | H | Cl | $C_2H_5$ | H | $-C_3H_7$-i | log p: 5.93 (pH = 7,5) |
| I-329 | F | F | Cl | $-CH_2-CH=CH_2$ | $-C_3H_7$-i | log p: 5.59 (pH = 7,5) |
| I-330 | F | F | $CH_3$ | H | 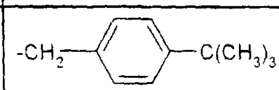 | Fp. 78-80°C |
| I-331 | F | F | $C_2H_5$ | H | $-C_4H_9$-n | Fp. 88-90°C |
| I-332 | F | F | $C_2H_5$ | H | 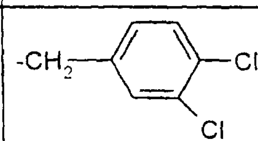 | Fp. 77-79°C |
| I-333 | H | Br | Cl | $-CH_2-CH=CH_2$ | 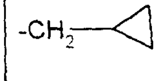 | log p: 6.30 (pH = 7.5) |
| I-334 | H | Br | Cl | $-CH_2-CH=CH_2$ | 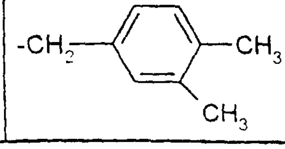 | log p: 7.16 (pH = 7,5) |

| Bsp.-Nr. | $X^1$ | $X^2$ | $R^1$ | $R^3$ | $R^2$ | physik. Charakter |
|---|---|---|---|---|---|---|
| | | | | | | |
| I-335 | H | Cl | $-CH_2-CH=CH_2$ | H | 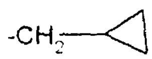 | log p: 5.96 (pH = 7,5) |
| I-336 | H | Cl | $-CH_2-CH=CH_2$ | H | 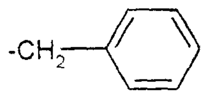 | log p: 5.98 (pH = 7,5) |
| I-337 | H | F | $-CH_2-CH=CH_2$ | H | $-C_3H_7$-i | log p: 5.62 (pH = 7,5) |
| I-338 | H | $CH_3$ | Cl | $-CH_2-CH=CH_2$ | $-C_3H_7$-n | log p: 6.93 (pH = 7,5) |
| I-339 | H | $CH_3$ | Cl | $-CH_2-CH=CH_2$ | $-CH_2-CH=CH_2$ | log p: 6.41 (pH = 7,5) |
| I-340 | F | F | $C_2H_5$ | H | 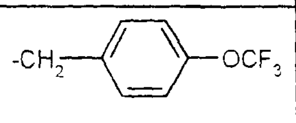 | Fp. 86-88°C |
| I-341 | H | Cl | $C_2H_5$ | H | 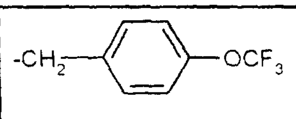 | Fp. 78-80°C |
| I-342 | F | F | Cl | Cl | $-CH(CH_3)_2$ | log p = 5.65 |
| I-343 | F | F | Cl | Cl | 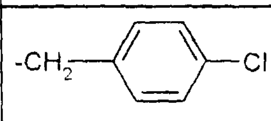 | Fp. 107°C |
| I-344 | F | F | Cl | Cl | $-CH_2-CH=CH_2$ | Fp. 93°C |
| I-345 | F | F | Cl | Cl | $-CF_3$ | Fp. 80°C |

## Herstellung von Ausgangsverbindungen

### Beispiel (VI-1)

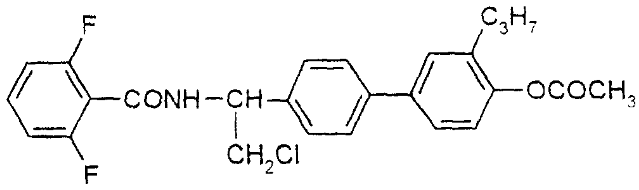

2,6-Difluor-N-[2-chlor-1-(4-(4-acetoxy-3-n-propylphenyl)phenyl)ethyl]-benzamid

Zu der Lösung von 14,9 g (59 mMol) 4-Acetoxy-3-n-propylbiphenyl und 16,2 g (65 mMol) 2,6-Difluor-N-[2-chlor-1-methoxyethyl]-benzamid in 100 ml Dichlormethan fügt man bei 0°C unter Rühren portionsweise 28,5 g (0,215 Mol) Aluminiumchlorid zu. Nach 30 Minuten Rührzeit werden nochmals 4 g (30 mMol) Aluminiumchlorid zugesetzt und 2 Stunden bei 0°C weitergerührt. Man gießt die Reaktionsmischung auf Eis, extrahiert das Produkt mit Dichlormethan und engt die organische Phase im Vakuum ein. Man erhält 27 g harziges Rohprodukt (log p = 3,92, pH = 2,3), welches ohne weitere Reinigung weiter umgesetzt wird (~ 100 % Rohausbeute)

**Beispiel (VI-2)**

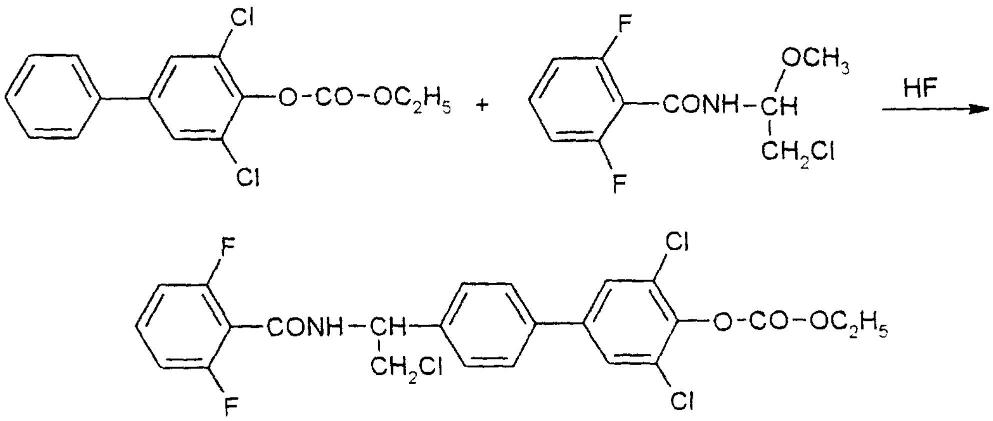


2,6-Difluor-N-[2-chlor-1-(4-(4-ethoxycarbonyl-3,5-dichlorphenyl)phenyl)ethyl]-benzamid

32,5 g (0,13 Mol) 2,6-difluor-N-[2chlor-1-methoxyethyl]-benzamidund 40 g (0,13 Mol) (2,6-dichlor-4-phenyl) phenylethyl-carbonat werden in 100 ml Dichlormethan gelöst und bei 0°C zu 500 ml wasserfreier Flußsäure gegeben. Nach dem Rühren über Nacht bei 14°C wird die Flußsäure im Vakuum entfernt, der Rückstand in Dichlormethan aufgenommen und mit Wasser gewaschen. Die org. Phase wird abgetrennt und einrotiert im Vakuum. Man erhält 54 g 2,6-difluor-N-[2-chlor-1-(4-(4-ethoxycarbonyl-3,5-dichlorphenyl)phenyl)ethylbenzamid als zähes Harz (log p (pH 2,3): 4, 17). Ohne weitere Einigung wird das Produkt weiter umgesetzt.

**Beispiel (II-1)**

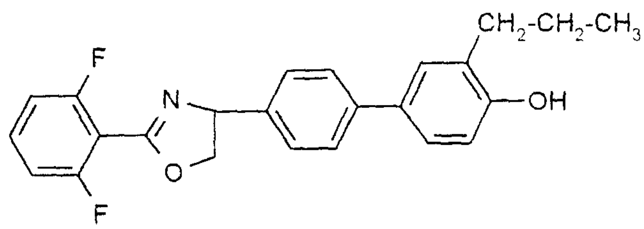

2-(2,6-Difluorphenyl)-4-(4-(4-hydroxy-3-n-propylphenyl)phenyl)-2-oxazolin

Zur Lösung von 27 g (57 mMol) 2,6-Difluor-N-[2-chlor-1-(4-(4-acetoxy-3-n-propylphenyl)phenyl)ethyl]-benzamid in 150 ml Dimethylformamid tropft man unter Rühren bei -10°C eine Lösung von 3,7 g (92 mMol) Natriumhydroxid in 10 ml Wasser. Nach dem Rühren über Nacht fügt man 43 ml 25 %ige Ammoniaklösung zu und läßt über Nacht rühren. Danach gießt man die Reaktionsmischung auf Eiswasser und extrahiert mehrmals mit Essigester. Die vereinigten Essigesterauszüge werden über $MgSO_4$ getrocknet und im Vakuum eingeengt.

Man erhält 21 g Rohprodukt (lg p = 3,77, pH = 7,5) (Rohausbeute 93,7 %), Eine kleine Probe des Rohproduktes mit Petrolether/Chloroform verrührt, lieferte ein Produkt von Fp. 210-215°C.

**Beispiel (II-30)**

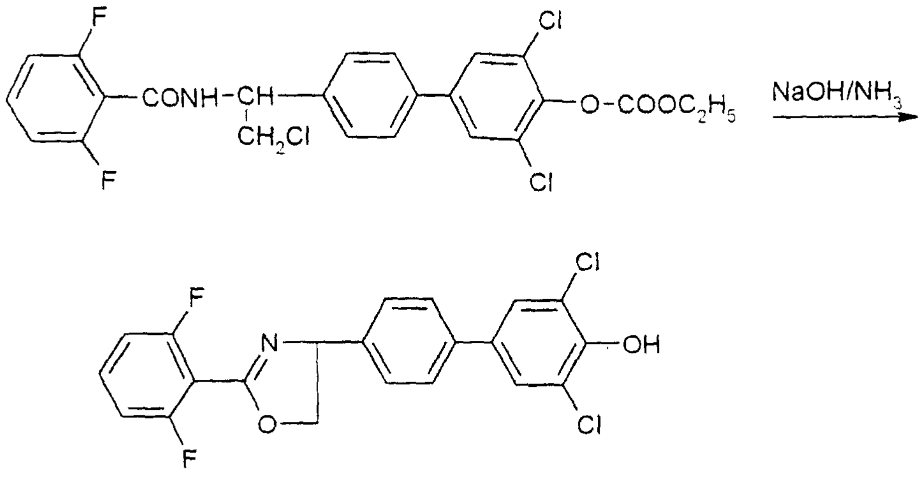


2-(2,6-Difluorphenyl)-4-(4-(4-hydroxy-3,5-dichlorphenyl)phenyl)-4,5-dihydrooxazolin

Zur Lösung von 53 g (0,1 Mol) 2,6-difluor-N-[2chlor-1-(4-(4-ethoxycarbonyl-3,5-dichlorphenyl)phenly)ethyl]-benzamid in 300 ml Dimethylformamid tropft man unter Rühren bei 10°C eine Lösung von 6 g (0,14 Mol) Natriumhydroxid in 10 ml Wasser zu. Nach einer Rührzeit von 5 h bei 10°C fügt man 100 ml 25 %ige Ammoniaklösung zu und rührt 3 Tage bei 20°C. Die Reaktionsmischung wird dann auf Wasser gegeben und das Produkt mit Dichlormethan extrahiert. Die org. Phase wird im Vakuum entfernt und das zurückbleibende Rohprodukt mit Diisopropylether verrührt. Man erhält 15 g (Ausbeute: 35,8 % d. Th.) 2-(2,6-Difluorphenyl)-4-(4-(4-hydroxy-3,5-dichlorphenyl)phenyl)-4,5-dihydro-oxazolin von Fp. 176-178°C.

Analog bzw. gemäß den allgemeinen Angaben zur Herstellung erhält man die folgenden Verbindungen der Formel (II)

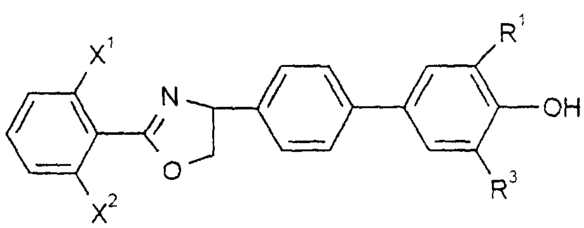

(II)

| Bsp.-Nr. | $X^1$ | $X^2$ | $R^1$ | $R^3$ | physik. Charakterisierung |
|---|---|---|---|---|---|
| II-2 | F | F | $CH_3$ | H | Fp. 186-188°C |
| II-3 | F | F | $CH_2CH=CH_2$ | H | Fp. 210-212°C |
| II-4 | H | Br | $CH_3$ | H | Fp. 163-164°C |
| II-5 | H | Cl | $-CH_2-CH_2-CH_3$ | H | Fp. 165-166°C |
| II-6 | H | Br | $-CH_2-CH_2-CH_3$ | H | Fp. 182-184°C |
| II-7 | Cl | F | Cl | H | Fp. 166°C |
| II-8 | Cl | F | $-CH_2-CH_2-CH_3$ | H | Fp. 210°C |

(fortgesetzt)

| | | | | | |
|---|---|---|---|---|---|
| II-9 | H | F | Cl | H | Fp. 138°C |
| II-10 | Cl | F | $CH_3$ | H | Fp. 190°C |
| II-11 | H | F | $-CH_2-CH_2-CH_3$ | H | Fp. 153°C |
| II-12 | F | F | $CH_3$ | $CH_2CH{=}CH_2$ | log p: 3,83 (pH 7,5) |
| II-13 | F | F | $CH_2CH{=}CH_2$ | $CH_2CH{=}CH_2$ | Fp. 108-10°C |
| II-14 | H | $CH_3$ | Cl | $CH_2CH-CH_2$ | log p: 4,75 (pH 7,5) |
| II-15 | H | $CH_3$ | $CH_3$ | H | |
| II-16 | H | $CH_3$ | Cl | H | Fp. 128-30°C |
| II-17 | H | Cl | $CH_3$ | H | Fp. 140-43°C |
| II-18 | H | Cl | $CH_2CH{=}CH_2$ | H | Fp. 85-88°C |
| II-19 | H | Cl | $CH_3$ | $CH_2CH-CH_2$ | log p: 4,14 (pH 7,5) |
| II-20 | H | Br | $CH_2CH{=}CH_2$ | $CH_2CH{=}CH_2$ | |
| II-21 | H | Br | Cl | $CH_2CH{=}CH_2$ | log p: 4,42 (pH 7,5) |
| II-22 | H | Br | Cl | H | Fp. 148-53°C |
| II-23 | H | $CH_3$ | $C_3H_7$-n | H | Fp. 139-41°C |
| II-24 | F | F | $C_2H_5$ | H | log p: 3,42 (pH 7,5) |
| II-25 | H | F | $CH_3$ | H | Fp. 136-38°C |
| II-26 | F | Cl | $-CH_2-CH{=}CH_2$ | H | Fp. 196°C |
| II-27 | H | Cl | $C_2H_5$ | H | Fp. 152-54°C |
| II-28 | H | F | $-CH_2-CH{=}CH_2$ | H | Fp. 134°C |
| II-29 | F | Cl | $C_2H_5$ | H | Fp. 193-95°C |
| II-30 | F | F | Cl | Cl | Fp. 176-178°C |
| II-31 | F | F | $CH_3$ | $CH_3$ | |

**Herstellung von 4-Acetoxy-3-ethyl-biphenyl**

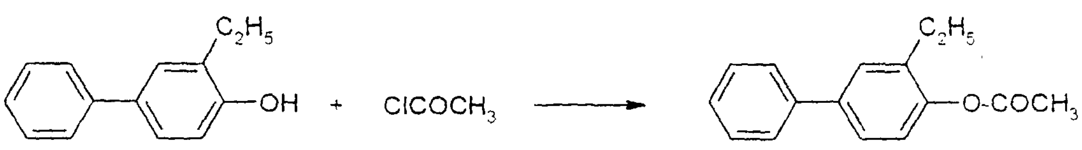

Zur Lösung von 7,3 g (37 mMol) 4-Hydroxy-3-ethyl-biphenyl und 3,2 g (40 mMol) Pyridin in 60 ml Chloroform tropft man 4.6 g (45 mMol) Acetanhydrid und kocht 3 Stunden am Rückfluß. Die erkaltete Lösung wird dann mit Wasser und verdünnter Salzsäure gewaschen, die organische Phase nach dem Trocknen eingedampft Man erhält 8,0 g (90 % Ausbeute d. Th.) 4-Acetoxy-3-ethyl-biphenyl als wasserklares Öl vom log p 3,92 (pH = 2,3).

**Herstellung von 4-Acetoxy-3-propyl-biphenyl**

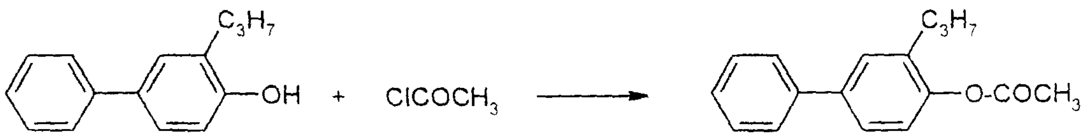

Zur Lösung von 10,6 g (50 mMol) 4-Hydroxy-3-propyl-biphenyl, 4,3 g (55 mMol) Pyridin und 0,1 g 4-(N,N-Dimethylamino)pyridin fügt man tropfenweise 6,1 g (60 mMol) Acetanhydrid und erwärmt 2 Stunden auf 50 bis 60°C Nach dem Stehen über Nacht bei 20°C wird die Reaktionslösung mit Wasser und verdünnter Salzsäure gewaschen und die organische Phase nach dem Trocknen eingeengt. Man erhält 11,4 g (Ausbeute: 89,8 % d. Th.) 4-Acetoxy-3-propyl-biphenyl als helles Öl vom log p = 4,32 (pH =2,3).

**Herstellung von (4-Phenyl-2-propyl-phenyl)-ethyl-carbonat**

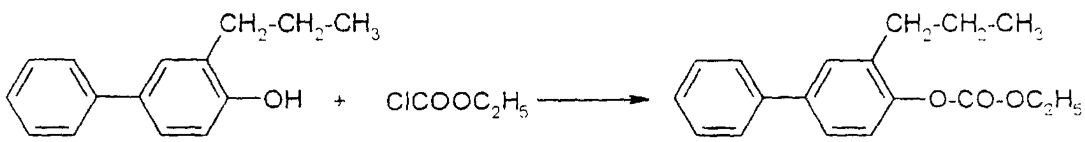

Zur Lösung von 36,3 g (0,17 Mol) 4-Hydroxy-3-propyl-biphenyl in 160 ml Essigester und 17,2 g (0,17 Mol) Triethylamin tropft man unter Kühlung bei 5 bis 10°C 18,4 g (0,17 Mol) Kohlensäureethylester-chlorid zu. Nach dem Stehen über Nacht bei 20°C wäscht man die Lösung mit Wasser und verdünnter Salzsäure und engt die organische Phase nach dem Trocknen ein. Man erhält 45 g (Ausbeute 93,2 % d. Th.) (4-Phenyl-2-propyl-phenyl)-ethyl-carbonat als helles Öl vom log p = 4,76 (pH = 2,3).

**Herstellung von (2,6-Dichlor-4-phenyl)phenyl-ethyl-carbonat**

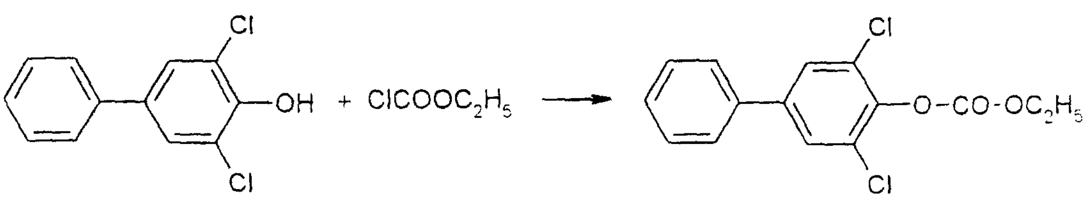

40,6 g (0,17 Mol) 3,5-dichlor-4-hydroxybiphenyl (hergestellt nach J.C. Colbert et. al. JACS 56, 202 (1934).) und 17,2 g (0,17 Mol) Triethylamin werden in 170 ml Essigester gelöst und bei 10°C tropfenweise mit 18,4 g (0,17 Mol) Chlorameisensäureethylester versetzt. Nach dem Rühren über Nacht wäscht man die Reaktionslösung mit Wasser und engt die organische Phase ein. Zur weiteren Reinigung wird das Rohprodukt (51,2 g) an Kieselgel im System Chloroform chromatographiert. Man erhält 48 g (Ausbeute 90,8 % d. Th.) von (2,6-Dichlor-4-phenyl-phenyl)-ethylcarbonat als Öl, log p (pH 7,5): 4,65.

Auf analoge Weise erhält man z.B.:

(2-Methyl-4-phenyl-phenyl)-ethyl-carbonat: log p = 4,04 (pH = 2,3)

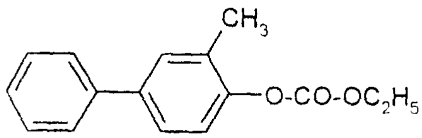

(2-Chlor-4-phenyl-phenyl)-ethyl-carbonat: log p = 4,16 (pH = 2,3)

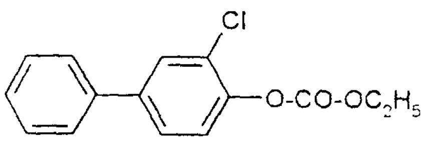

**Anwendungsbeispiele**

**Beispiel A**

**Myzus-Test**

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

In diesem Test bewirkten z.B. die Verbindungen gemäß den Herstellungsbeispielen I-19, I-24, I-30 und I-31 bei einer beispielhaften Wirkstoffkonzentration von 0,01 % eine Abtötung von jeweils mindestens 90 % nach 6 Tagen.

**Beispiel B**

**Phaedon-Larven-Test**

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man l Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Larven des Meerrettichkäfers (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käferlarven abgetötet wurden.

In diesem Test bewirkten z.B. die Verbindungen gemäß den Herstellungsbeispielen I-2, I-19, I-20, I-30 und I-31 bei einer beispielhaften Wirkstoffkonzentration von 0,01 % jeweils eine Abtötung von 100 % nach 7 Tagen.

Die Verbindungen gemäß den Herstellungsbeispielen 1-342, I-343 und I-344 bewirkten in diesem Test bei einer beispielhaften Wirkstoffkonzentration von 0,1 % jeweils eine Abtötung von 100 % nach 7 Tagen.

**Beispiel C**

**Spodoptera-Test**

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß den Herstellungsbeispielen I-2, I-19, I-21, I-23, I-25 und I-30 bei einer beispielhaften Wirkstoffkonzentration von 0,0001 % eine Abtötung von jeweils mindestens 90 % nach 7 Tagen.

Die Verbindungen gemäß den Herstellungsbeispielen 1-342, 1-343 und 1-344 bewirkten in diesem Test bei einer beispielhaften Wirkstoffkonzentration von 0,1 % jeweils eine Abtötung von 100 % nach 7 Tagen.

**Beispiel D**

**Tetranychus-Test (OP-resistent/Tauchbehandlung)**

Lösungsmittel: 3 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaligem Wasser auf die gewünschte Konzentration.

Bohnenpflanzen (Phaseolus vulgaris), die stark von allen Stadien der gemeinen Spinnmilbe Tetranychus urticae befallen sind, werden in eine Wirkstoffzubereitung der gewünschten Konzentration getaucht.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test hatten z.B. die Verbindungen gemäß den Herstellungsbeispielen 1-2, I-19, I-21, I-24, I-25, I-27, I-30 und I-31 bei einer beispielhaften Wirkstoffkonzentration von 0,0001 % eine Wirkung von jeweils mindestens 98 % nach 13 Tagen.

Die Verbindungen gemäß den Herstellungsbeispielen I-342, I-343 und I-344 bewirkten in diesem Test bei einer beispielhaften Wirkstoffkonzentration von 0,01 % eine Abtötung von 100 % nach 7 Tagen.

**Beispiel E**

**Test mit Fliegenlarven / Entwicklungshemmende Wirkung**

Testtiere: Alle larvalen Stadien von Lucilia cuprina (OP-resistent) [Puppen und Adulte (ohne Kontakt zum Wirkstoff)]

Lösungsmittel: 35 Gewichtsteile Ethylenglykolmonomethylether 35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

30-50 Larven je Konzentration werden auf in Glasröhrchen befindliches Pferdefleisch (1 $cm^3$) gebracht, auf welches 500 µl der zu testenden Verdünnung pipettiert werden. Die Glasröhrchen werden in Kunststoffbecher gestellt, deren Boden mit Seesand bedeckt ist, und im klimatisierten Raum (26°C ± 1,5°C, 70 % rel. Feuchte ± 10 %) aufbewahrt. Die Wirkungskontrolle erfolgt nach 24 Stunden und 48 Stunden (larvizide Wirkung). Nach dem Auswandern der Larven (ca. 72 h) werden die Glasröhrchen entfernt und gelochte Kunststoffdeckel auf die Becher gesetzt. Nach 1½-facher Entwicklungsdauer (Schlupf der Kontrollfliegen) werden die geschlüpften Fliegen und die Puppen/Puppenhüllen ausgezählt.

Als Kriterium für die Wirkung gilt der Eintritt des Todes bei den behandelten Larven nach 48 h (larvizider Effekt), bzw. die Hemmung des Adultschlupfes aus den Puppen bzw. die Hemmung der Puppenbildung. Als Kriterium für die in-vitro-Wirkung einer Substanz gilt die Hemmung der Flohentwicklung, bzw. ein Entwicklungsstillstand vor dem Adulten-Stadium. Dabei bedeutet 100 % larvizide Wirkung, daß nach 48 Stunden alle Larven abgestorben sind. 100 % entwicklungsinhibitorische Wirkung bedeutet, daß keine adulte Fliegen geschlüpft sind.

**Patentansprüche**

**1.** Verbindungen der Formel (I)

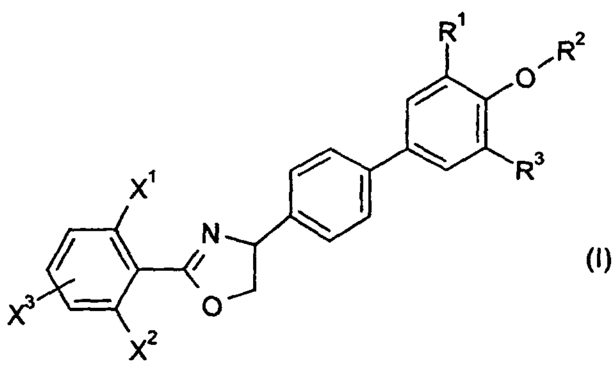

(I)

in welcher

$X^1$ für Wasserstoff, Fluor, Chlor, Brom, Iod, $C_1$-$C_6$-Alkyl, $C_1$-$C_6$-Alkoxy oder $C_1$-$C_6$-Alkylthio steht;

$X^2$ für Fluor, Chlor, Brom, Iod, $C_1$-$C_6$-Alkyl, $C_1$-$C_6$-Alkoxy oder $C_1$-$C_6$-Alkylthio steht;

$X^3$ für Wasserstoff, Fluor, Chlor, $C_1$-$C_6$-Alkyl oder $C_1$-$C_6$-Alkoxy steht;

$R^1$ für Wasserstoff, Fluor, Chlor, Brom, $C_1$-$C_6$-Alkyl oder $-CH_2-CR^4=CH_2$ steht;

$R^2$ für $C_1$-$C_8$-Alkyl, $C_1$-$C_6$-Hydroxyalkyl, $C_3$-$C_{10}$-Alkenyl, $C_3$-$C_{12}$-Alkinyl, für jeweils gegebenenfalls durch Ha-

logen oder $C_1$-$C_4$-Alkyl substituiertes $C_3$-$C_6$-Cycloalkyl oder $C_4$-$C_6$-Cycloalkenyl, für gegebenenfalls durch Halogen, $C_1$-$C_4$-Alkyl, $C_2$-$C_4$-Alkenyl, $C_2$-$C_4$-Halogenalkenyl, Phenyl, Halogenphenyl, Styryl oder Halogenstyryl substituiertes $C_3$-$C_6$-Cycloalkyl-$C_1$-$C_4$-alkyl, für gegebenenfalls durch Halogen oder $C_1$-$C_4$-Alkyl substituiertes $C_4$-$C_8$-Cycloalkenyl-$C_1$-$C_2$-alkyl, oder fiir gegebenenfalls einfach bis vierfach, gleich oder verschieden durch Nitro, Halogen, $C_1$-$C_{12}$-Alkyl, $C_1$-$C_{12}$-Halogenalkyl, $C_1$-$C_{12}$-Alkylthio, $C_1$-$C_{12}$-Halogenalkylthio, $C_1$-$C_{12}$-Alkoxy oder $C_1$-$C_{12}$-Halogenalkoxy substituiertes Phenyl-$C_1$-$C_4$-alkyl, für Naphthyl-$C_1$-$C_3$-alkyl oder Tetrahydronaphthyl-$C_1$-$C_3$-alkyl steht;

$R^3$ fiir Wasserstoff $C_1$-$C_4$-Alkyl, Fluor, Chlor, Brom oder für $-CH_2-CR^4=CH_2$, steht;

$R^4$ für Wasserstoff, $C_1$-$C_{12}$-Alkyl oder fiir gegebenenfalls einfach bis vierfach, gleich oder verschieden durch Halogen, $C_1$-$C_{12}$-Alkyl, $C_1$-$C_{12}$-Halogenalkyl, $C_1$-$C_{12}$-Alkoxy oder $C_1$-$C_{12}$-Halogenalkoxy substituiertes Phenyl steht;

wobei $R^1$ und $R^3$ nicht gleichzeitig für Wasserstoff stehen.

**2.** Verbindungen der Formel (I), gemäß Anspruch 1, in welchen

$X^1$ für Wasserstoff, Fluor oder Chlor steht;

$X^2$ für Fluor, Chlor, Brom, Iod, $C_1$-$C_3$-Alkyl oder $C_1$-$C_3$-Alkoxy steht;

$X^3$ für Wasserstoff, Fluor, Chlor, $C_1$-$C_3$-Alkyl oder $C_1$-$C_3$Alkoxy steht;

$R^1$ für Wasserstoff, Fluor, Chlor, Brom, $C_1$-$C_4$-Alkyl oder $-CH_2-CR^4=CH_2$ steht;

$R^2$ für $C_1$-$C_6$-Alkyl, $C_1$-$C_4$-Hydroxyalkyl, $C_3$-$C_{10}$-Alkenyl, $C_3$-$C_5$-Alkinyl, für jeweils gegebenenfalls durch Halogen oder $C_1$-$C_4$-Alkyl substituiertes Cyclohexyl oder $C_4$-$C_6$-Cycloalkenyl, für gegebenenfalls durch Halogen, $C_1$-$C_4$-Alkyl, $C_2$-$C_4$-Alkenyl, $C_2$-$C_3$-Halogenalkenyl, Phenyl, Halogenphenyl, Styryl oder Halogenstyryl substituiertes $C_3$-$C_6$-Cycloalkyl-$C_1$-$C_4$-alkyl, für gegebenenfalls durch Halogen substituiertes $C_4$-$C_6$-Cycloalkenyl-methyl oder für gegebenenfalls einfach oder zweifach, gleich oder verschieden durch Nitro, Halogen, $C_1$-$C_4$-Alkyl, $C_1$-$C_4$-Halogenalkyl, $C_1$-$C_4$-Alkylthio, $C_1$-$C_4$-Halogenalkylthio, $C_1$-$C_4$-Alkoxy oder $C_1$-$C_4$-Halo-genalkoxy substituiertes Benzyl, für Naphthylmethyl oder Tetrahydro-naphthylmethyl steht;

$R^3$ für Wasserstoff, $C_1$-$C_3$-Alkyl, Chlor, Brom oder oder $-CH_2-CR^4=CH_2$ steht.

$R^4$ fiir Wasserstoff, $C_1$-$C_4$-Alkyl oder für gegebenenfalls einfach oder zweifach, gleich oder verschieden durch Fluor, Chlor, Brom, $C_1$-$C_4$-Alkyl, $C_1$-$C_4$-Halogenalkyl oder $C_1$-$C_4$-Halogenalkoxy substituiertes Phenyl steht;

wobei $R^1$ und $R^3$ nicht gleichzeitig für Wasserstoff stehen.

**3.** Verbindungen der Formel (I), gemäß Anspruch 1, in welchen

$X^1$ für Wasserstoff, Fluor oder Chlor steht;

$X^2$ für Fluor, Chlor, Brom, Iod, Methyl oder Methoxy steht;

$X^3$ für Wasserstoff, Fluor, Chlor oder Methyl.

$R^1$ für Wasserstoff, Fluor, Chlor, Brom, Methyl, Ethyl, n- oder i-Propyl oder Allyl steht.

$R^2$ für Methyl, Ethyl, n- oder i-Propyl, n-Butyl, i-Butyl, s-Butyl, n-Pentyl, n-Hexyl, Cyclohexyl, Hydroxyethyl, Hydroxypropyl, Hydroxybutyl, $-CH_2-CH=CH_2$, $-CH_2-C(CH_3)=CH_2$, $-CH_2-C\equiv CH$, $CH(CH_3)C\equiv CH$, für eine der Cycloalkylalkylgruppierungen:

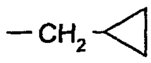 , 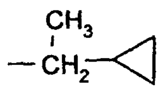 , 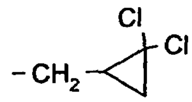 , 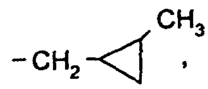 ,

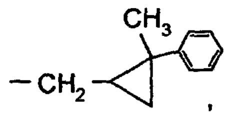 , 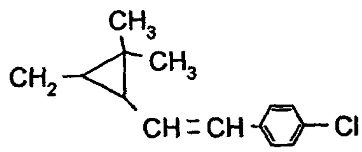 , 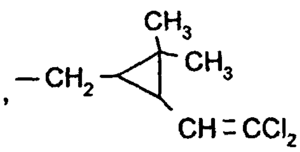 ,

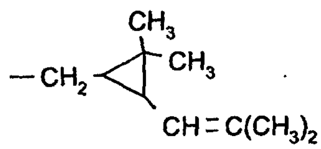 ;

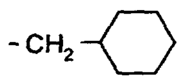 , 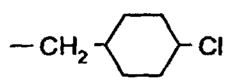 , 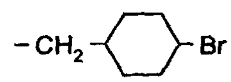 ,

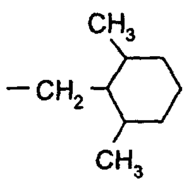 , 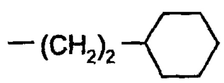 , 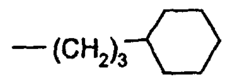 ,

für

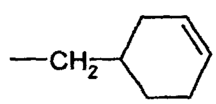 ,

für einen der folgenden Reste:

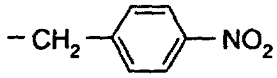 , 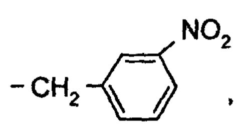 , 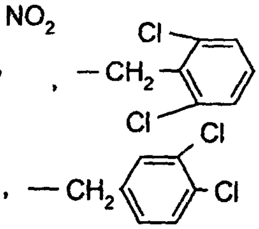 ,

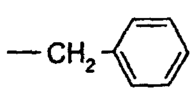 , 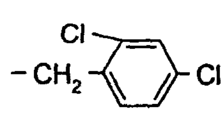 , ,

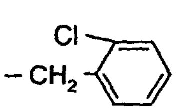 , 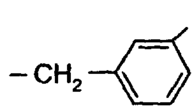 , 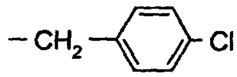 ,

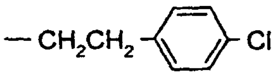 , 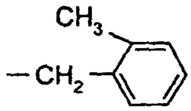 , 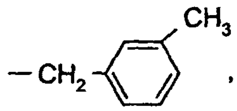 ,

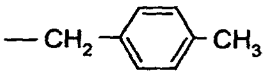 , 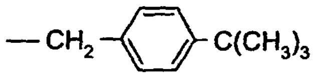 ,

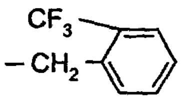 , 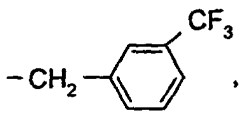 , 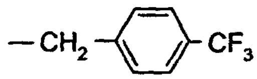 ,

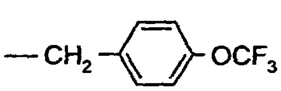 , 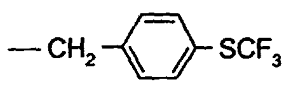 , 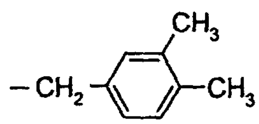 ,

oder für

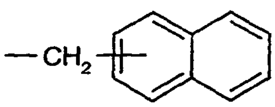 oder 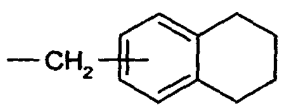 steht;

$R^3$ für Wasserstoff, Chlor, Brom, Methyl oder $-CH_2-CH=CH_2$, insbesondere fiir Wasserstoff, Chlor oder $-CH_2-CH=CH_2$ steht, wobei $R^1$ und $R^3$ nicht gleichzeitig für Wasserstoff stehen.

**4.** Verfahren zur Herstellung von Verbindungen der Formel (I) gemäß Anspruch 1 bis 3 und der Formel (Ia) gemäß Anspruch 5 und 6,
**dadurch gekennzeichnet, dass** man

A) Verbindungen der Formel (II)

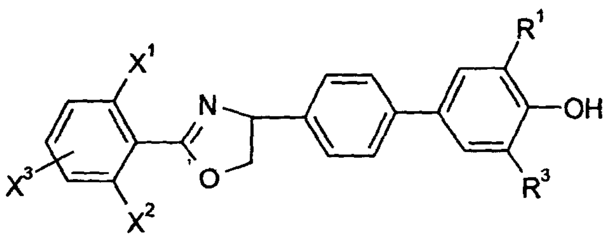 (II),

in welcher

$X^1$, $X^2$, $X^3$, $R^3$ und $R^1$ die in Anspruch 1 angegebenen Bedeutungen haben, wobei hier $R^1$ = $R^3$ = Wasserstoff möglich ist.
mit Verbindungen der Formel (III)

$$Z - R^2 \qquad (III),$$

in welcher

$R^2$ die in Anspruch 1 angegebene Bedeutung hat und

Z fiir eine Abgangsgruppe steht,

gegebenenfalls in Gegenwart eines Verdünnungsmittels und gegebenenfalls in Gegenwart einer Base umsetzt und

B) gegebenenfalls anschließend die so für $R^2$ = $-CH_2-CR^4=CH_2$ und $R^3$ = H erhaltenen Verbindungen der Formel (IV)

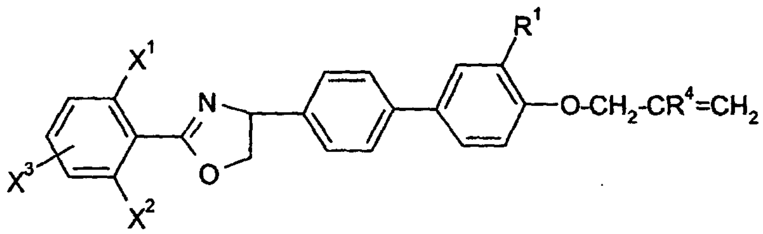

(IV),

in welcher
$R^1$, $R^4$, $X^1$, $X^2$ und $X^3$ die in Anspruch 1 angegebenen Bedeutungen haben,
einer Claisen-Umlagerung unterwirft und

C) gegebenenfalls anschließend die so erhaltenen Verbindungen der Formel (V)

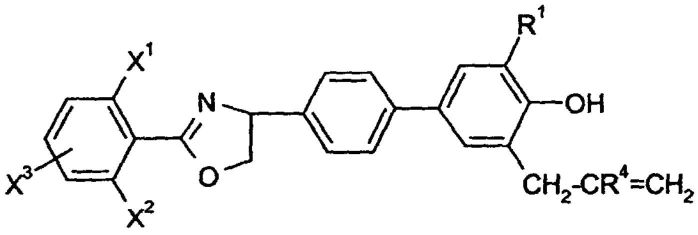

(V),

in welcher
$X^1$, $X^2$, $X^3$, $R^1$ und $R^4$ die oben angegebenen Bedeutungen haben,
mit Verbindungen der Formel (III)

$$Z - R^2 \qquad (III),$$

in welcher
$R^2$ und Z die oben angegebenen Bedeutungen haben,

gegebenenfalls in Gegenwart eines Verdünnungsmittels und gegebenenfalls in Gegenwart einer Base umsetzt und gegebenenfalls anschließend für $R^2$ = $-CH_2-CR^4=CH_2$ und $R^1$ = H die Schritte B) und C) wiederholt, wobei man Verbindungen der Formel (Va) erhält

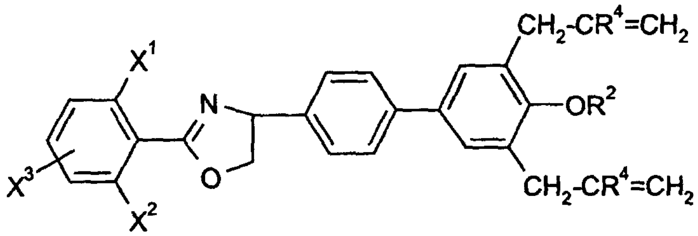

(Va),

in welcher
$X^1$, $X^2$, $X^3$, $R^2$ und $R^4$ die oben angegebene Bedeutung haben,
und die Reste $R^4$ gleich oder verschieden sein können.

**5.** Verbindungen der Formel (Ia)

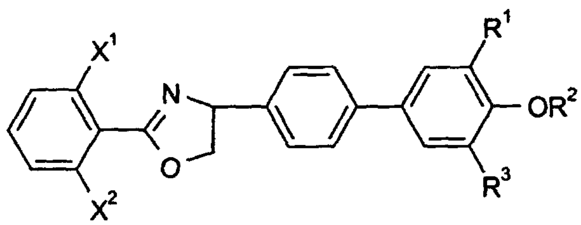

(Ia),

in welchen
$R^1$, $R^2$, $R^3$, $X^1$ und $X^2$ die in Anspruch 1 genannten Bedeutungen haben.

**6.** Verbindungen der Formel (Ia) gemäß Anspruch 5
in welcher

$X^1$ für Wasserstoff oder Fluor steht.

$X^2$ für Fluor, Chlor, Brom oder Methyl steht.

$R^1$ für Chlor, Methyl, Ethyl, n- oder i-Propyl oder Allyl steht und

$R^2$ die in Anspruch 1 genannten Bedeutungen hat.

$R^3$ die in Anspruch 1 genannten Bedeutungen hat.

**7.** Schädlingsbekämpfungsmittel, **gekennzeichnet durch** einen Gehalt an mindestens einer Verbindung der Formel (I) gemäß Anspruch 1.

**8.** Verwendung von Verbindungen der Formel (I) gemäß Anspruch 1 zur Bekämpfung von Schädlingen.

**9.** Verfahren zur Bekämpfung von Schädlingen, **dadurch gekennzeichnet, dass** man Verbindungen der Formel (I) gemäß Anspruch 1 auf Schädlinge und/oder ihren Lebensraum einwirken läßt.

**10.** Verfahren zur Herstellung von Schädlingsbekämpfungsmitteln, **dadurch gekennzeichnet, dass** man Verbindungen der Formel (I) gemäß Anspruch 1 mit Streckmitteln und/oder oberflächenaktiven Mitteln vermischt.

**11.** Verwendung von Verbindungen der Formel (I) gemäß Anspruch 1 zur Herstellung von Schädlingsbekämpfungsmitteln.

## Claims

**1.** Compounds of the formula (I)

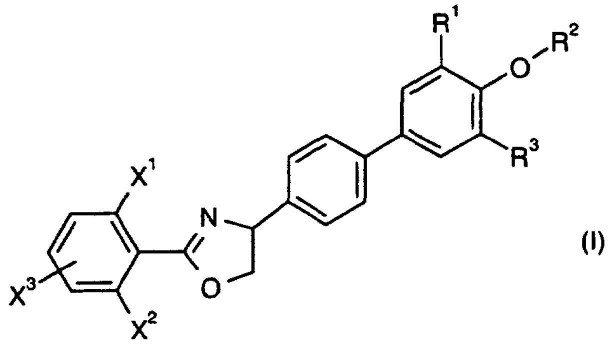

(I)

in which

$X^1$ represents hydrogen, fluorine, chlorine, bromine, iodine, $C_1$-$C_6$-alkyl, $C_1$-$C_6$-alkoxy or $C_1$-$C_6$-alkylthio;

$X^2$ represents fluorine, chlorine, bromine, iodine, $C_1$-$C_6$-alkyl, $C_1$-$C_6$-alkoxy or $C_1$-$C_6$-alkylthio;

$X^3$ represents hydrogen, fluorine, chlorine, $C_1$-$C_6$-alkyl or $C_1$-$C_6$-alkoxy;

$R^1$ represents hydrogen, fluorine, chlorine, bromine, $C_1$-$C_6$-alkyl or $-CH_2-CR^4=CH_2$;

$R^2$ represents $C_1$-$C_8$-alkyl, $C_1$-$C_6$-hydroxyalkyl, $C_3$-$C_{10}$-alkenyl, $C_3$-$C_{12}$-alkinyl, represents respectively optionally halogen- or $C_1$-$C_4$-alkyl-substituted $C_3$-$C_6$-cycloalkyl or $C_4$-$C_6$-cycloalkenyl, represents optionally halogen-, $C_1$-$C_4$-alkyl-, $C_2$-$C_4$-alkenyl-, $C_2$-$C_4$-halogenoalkenyl-, phenyl-, halogenophenyl-, styryl- or halogenostyryl-substituted $C_3$-$C_6$-cycloalkyl-$C_1$-$C_4$-alkyl, represents optionally halogen- or $C_1$-$C_4$-alkyl-substituted $C_4$-$C_8$-cycloalkenyl-$C_1$-$C_2$-alkyl,
or represents phenyl-$C_1$-$C_4$-alkyl which is optionally mono- to tetrasubstituted by identical or different substituents from the group consisting of nitro, halogen, $C_1$-$C_{12}$-alkyl, $C_1$-$C_{12}$-halogenoalkyl, $C_1$-$C_{12}$-alkylthio, $C_1$-$C_{12}$-halogenoalkylthio, $C_1$-$C_{12}$-alkoxy and $C_1$-$C_{12}$-halogenoalkoxy,
represents naphthyl-$C_1$-$C_3$-alkyl or tetrahydronaphthyl-$C_1$-$C_3$-alkyl;

$R^3$ represents hydrogen, $C_1$-$C_4$-alkyl, fluorine, chlorine, bromine or represents $-CH_2-CR^4=CH_2$;

$R^4$ represents hydrogen, $C_1$-$C_{12}$-alkyl or
represents phenyl which is optionally mono- to tetrasubstituted by identical or different substituents from the group consisting of halogen, $C_1$-$C_{12}$-alkyl, $C_1$-$C_{12}$-halogenoalkyl, $C_1$-$C_{12}$-alkoxy and $C_1$-$C_{12}$-halogenoalkoxy;

where $R^1$ and $R^3$ do not simultaneously represent hydrogen.

**2.** Compounds of the formula (I) according to Claim 1 in which

$X^1$ represents hydrogen, fluorine or chlorine;

$X^2$ represents fluorine, chlorine, bromine, iodine, $C_1$-$C_3$-alkyl or $C_1$-$C_3$-alkoxy;

$X^3$ represents hydrogen, fluorine, chlorine, $C_1$-$C_3$-alkyl or $C_1$-$C_3$-alkoxy;

$R^1$ represents hydrogen, fluorine, chlorine, bromine, $C_1$-$C_4$-alkyl or -$CH_2$-$CR^4$=$CH_2$;

$R^2$ represents $C_1$-$C_6$-alkyl, $C_1$-$C_4$-hydroxyalkyl, $C_3$-$C_{10}$-alkenyl, $C_3$-$C_5$-alkinyl, represents respectively optionally halogen- or $C_1$-$C_4$-alkyl-substituted cyclohexyl or $C_4$-$C_6$-cycloalkenyl, represents optionally halogen-, $C_1$-$C_4$-alkyl-, $C_2$-$C_4$-alkenyl-, $C_2$-$C_3$-halogenoalkenyl-, phenyl-, halogenophenyl-, styryl- or halogenostyryl-substituted $C_3$-$C_6$-cycloalkyl-$C_1$-$C_4$-alkyl, represents optionally halogen-substituted $C_4$-$C_6$-cycloalkenylmethyl or
represents benzyl which is optionally mono- or disubstituted by identical or different substituents from the group consisting of nitro, halogen, $C_1$-$C_4$-alkyl, $C_1$-$C_4$-halogenoalkyl, $C_1$-$C_4$-alkylthio, $C_1$-$C_4$-halogenoalkylthio, $C_1$-$C_4$-alkoxy and $C_1$-$C_4$-halogenoalkoxy, represents naphthylmethyl or tetrahydronaphthylmethyl;

$R^3$ represents hydrogen, $C_1$-$C_3$-alkyl, chlorine, bromine or -$CH_2$-$CR^4$=$CH_2$;

$R^4$ represents hydrogen, $C_1$-$C_4$-alkyl or represents phenyl which is optionally mono- or disubstituted by identical or different substituents from the group consisting of fluorine, chlorine, bromine, $C_1$-$C_4$-alkyl, $C_1$-$C_4$-halogenoalkyl and $C_1$-$C_4$-halogenoalkoxy;

where $R^1$ and $R^3$ do not simultaneously represent hydrogen.

**3.** Compounds of the formula (I) according to Claim 1 in which

$X^1$ represents hydrogen, fluorine or chlorine;

$X^2$ represents fluorine, chlorine, bromine, iodine, methyl or methoxy;

$X^3$ represents hydrogen, fluorine, chlorine or methyl;

$R^1$ represents hydrogen, fluorine, chlorine, bromine, methyl, ethyl, n-or i-propyl or represents allyl;

$R^2$ represents methyl, ethyl, n- or i-propyl, n-butyl, i-butyl, s-butyl, n-pentyl, n-hexyl, cyclohexyl, hydroxyethyl, hydroxypropyl, hydroxybutyl, -$CH_2$-CH=$CH_2$, -$CH_2$-C($CH_3$)=$CH_2$, -$CH_2$-C≡CH, -CH($CH_3$)C≡CH, represents one of the cycloalkylalkyl groupings:

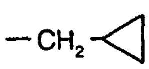 , 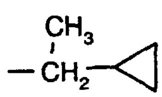 , 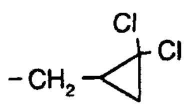 , 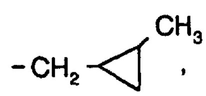

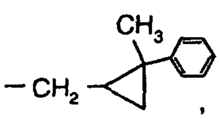 , 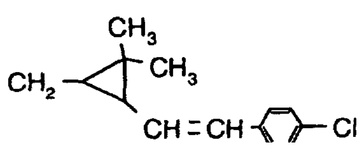 , 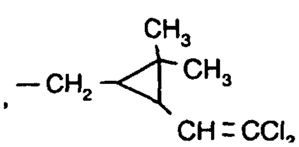 ,

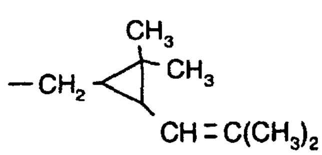 ;

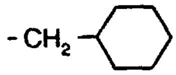 , 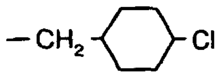 , 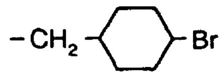 ,

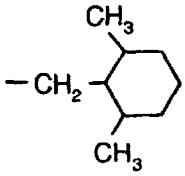 , 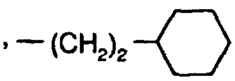 , 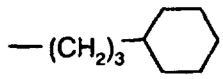 ,

represents

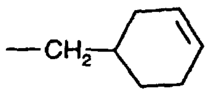 ,

represents one of the radicals below:

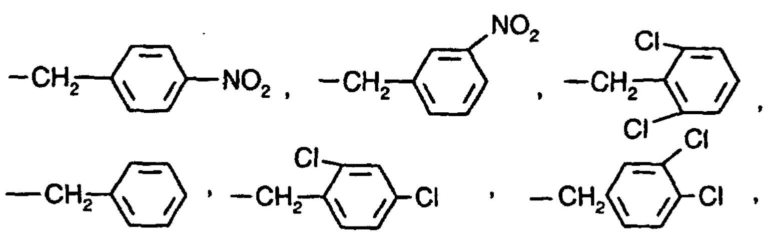

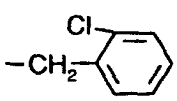 , 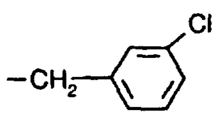 , 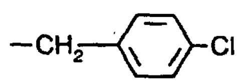 ,

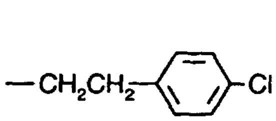 , 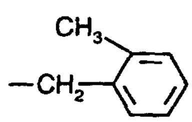 , 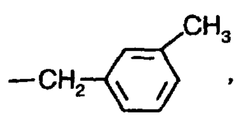 ,

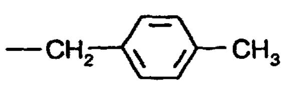 , 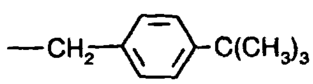 ,

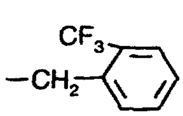 , 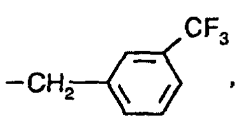 , 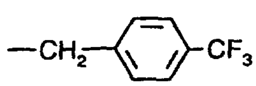 ,

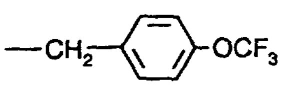 , 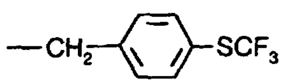 , 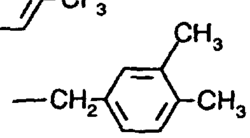 ,

or represents

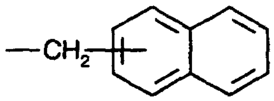

or

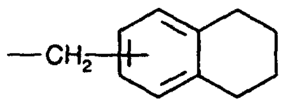

;

$R^3$ represents hydrogen, chlorine, bromine, methyl or $-CH_2-CH=CH_2$, in particular hydrogen, chlorine or $-CH_2-CH=CH_2$, where $R^1$ and $R^3$ do not simultaneously represent hydrogen.

**4.** Process for preparing compounds of the formula (I) according to Claims 1 to 3 and of the formula (Ia) according to Claims 5 and 6,

**characterized in that**

A) compounds of the formula (II)

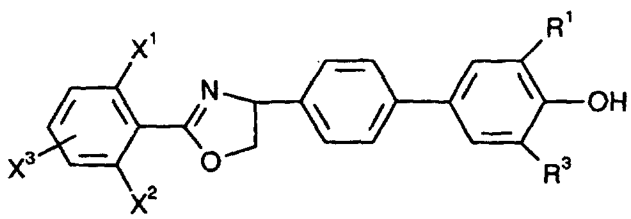

(II),

in which
$X^1$, $X^2$, $X^3$, $R^3$ and $R^1$ are each as defined in Claim 1, $R^1 = R^3$ = hydrogen being possible here, are reacted with compounds of the formula (III)

$$Z - R^2 \qquad \text{(III)},$$

in which

$R^2$ is as defined in Claim 1 and

Z represents a leaving group,

if appropriate in the presence of a diluent and if appropriate in the presence of a base and

B) if appropriate, the compounds of the formula (IV) obtained in this manner for $R^2 = -CH_2-CR^4=CH_2$ and $R^3$ = H

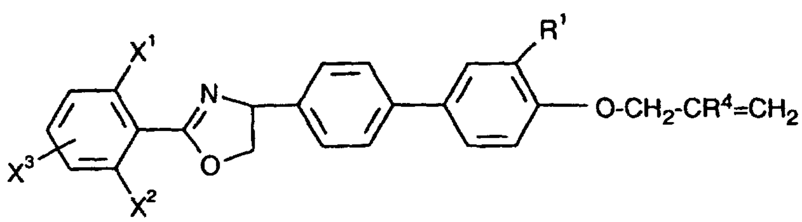

(IV),

in which
$R^1$, $R^4$, $X^1$, $X^2$ and $X^3$ are each as defined in Claim 1, are subsequently subjected to a Claisen rearrangement and

C) if appropriate, the resulting compounds of the formula (V)

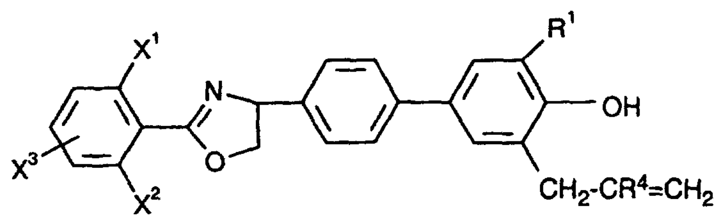

(V),

in which
$X^1$, $X^2$, $X^3$, $R^1$ and $R^4$ are each as defined above,
are subsequently reacted with compounds of the formula (III)

$$Z - R^2 \quad \text{(III)},$$

in which
$R^2$ and Z are each as defined above,
if appropriate in the presence of a diluent and if appropriate in the presence of a base and,
if appropriate, steps B) and C) are then repeated for $R^2$ = $-CH_2-CR^4=CH_2$ and $R^1$ = H, affording compounds of formula (Va)

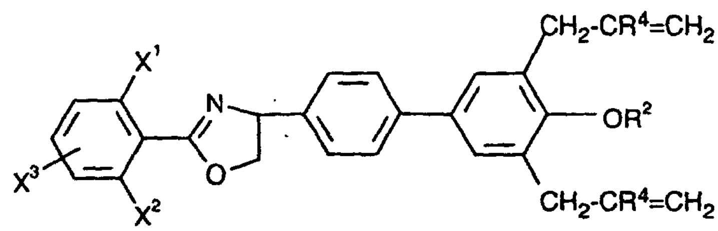

(Va),

in which
$X^1$, $X^2$, $X^3$, $R^2$ and $R^4$ are each as defined above,
and the radicals $R^4$ may be identical or different.

**5.** Compounds of the formula (Ia)

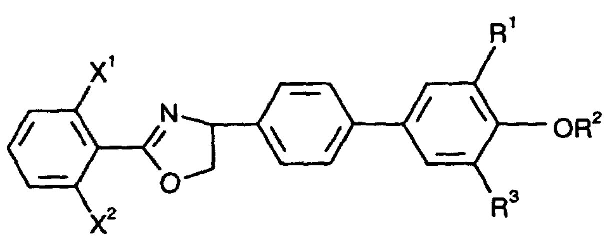

(Ia),

in which
$R^1$, $R^2$, $R^3$, $X^1$ and $X^2$ are each as defined in Claim 1.

**6.** Compounds of the formula (Ia) according to Claim 5
in which

$X^1$ represents hydrogen or fluorine;
$X^2$ represents fluorine, chlorine, bromine or methyl;

$R^1$ represents chlorine, methyl, ethyl, n- or i-propyl or allyl and
$R^2$ is as defined in Claim 1;
$R^3$ is as defined in Claim 1.

**7.** Pesticides, **characterized in that** they contain at least one compound of the formula (I) according to Claim 1.

**8.** Use of compounds of the formula (I) according to Claim 1 for controlling pests.

**9.** Method for controlling pests, **characterized in that** compounds of the formula (I) according to Claim 1 are allowed to act on pests and/or their habitat.

**10.** Process for preparing pesticides, **characterized in that** compounds of the formula (I) according to Claim 1 are mixed with extenders and/or surfactants.

**11.** Use of compounds of the formula (I) according to Claim 1 for preparing pesticides.

**Revendications**

**1.** Composés de la formule (I) :

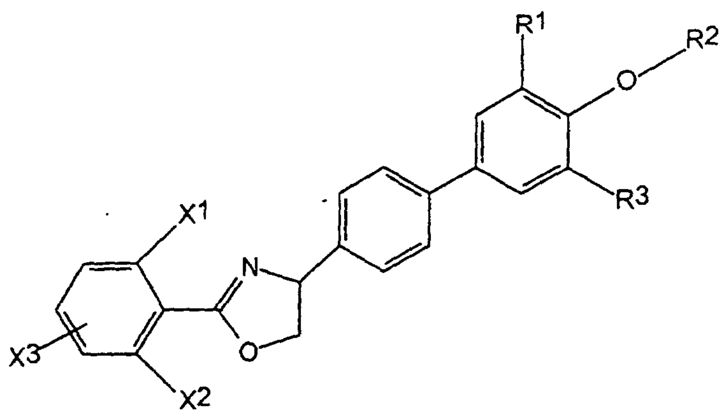

(I)

dans laquelle :

$X^1$ représente l'atome d'hydrogène, de fluor, de chlore, de brome, d'iode, un radical alcoyle en $C_1$-$C_6$, alcoxy en $C_1$-$C_6$ ou alcoylthio en $C_1$-$C_6$ ;
$X^2$ représente l'atome de fluor, de chlore, de brome, d'iode, un radical alcoyle en $C_1$-$C_6$, alcoxy en $C_1$-$C_6$ ou alcoylthio en $C_1$-$C_6$ ;
$X^3$ représente l'atome d'hydrogène, de fluor, de chlore, un radical alcoyle en $C_1$-$C_6$ ou alcoxy en $C_1$-$C_6$ ;
$R^1$ représente l'atome d'hydrogène, de fluor, de chlore, de brome, un radical alcoyle en $C_1$-$C_6$, ou le radical $-CH_2-CR^4=CH_2$ ;
$R^2$ représente un radical alcoyle en $C_1$-$C_8$, hydroxyalcoyle en $C_1$-$C_6$, alcényle en $C_3$-$C_{10}$, alcynyle en $C_3$-$C_{12}$, un radical cycloalcoyle en $C_3$-$C_6$ ou cycloalcényle en $C_4$-$C_6$, chaque fois le cas échéant substitué par un atome d'halogène ou par un radical alcoyle en $C_1$-$C_4$, représente un radical (cycloalcoyl en $C_3$-$C_6$)alcoyle en $C_1$-$C_4$, le cas échéant substitué par un atome d'halogène, par un radical alcoyle en $C_1$-$C_4$, alcényle en $C_2$-$C_4$, halogénoalcényle en $C_2$-$C_4$, phényle, halogénophényle, styryle ou halogénostyryle, représente un radical (cycloalcényl en $C_4$-$C_8$)alcoyle en $C_1$-$C_2$, le cas échéant substitué par un atome d'halogène ou par un radical alcoyle en $C_1$-$C_4$, ou représente un radical phényl(alcoyle en $C_1$-$C_4$), le cas échéant substitué une à quatre fois, de manière identique ou différente, par le radical nitro, un atome d'halogène, un radical alcoyle en $C_1$-$C_{12}$, halogénoalcoyle en $C_1$-$C_{12}$, alcoylthio en $C_1$-$C_{12}$, halogénoalcoylthio en $C_1$-$C_{12}$, alcoxy en $C_1$-$C_{12}$ ou halogénoalcoxy en $C_1$-$C_{12}$, représente un radical naphtyl(alcoyle en $C_1$-$C_3$) ou tétrahydronaphtyl(alcoyle en $C_1$-$C_3$) ;
$R^3$ représente l'atome d'hydrogène, un radical alcoyle en $C_1$-$C_4$, l'atome de fluor, de chlore, de brome, ou le

radical $-CH_2-CR^4=CH_2$ ;
$R^4$ représente l'atome d'hydrogène, un radical alcoyle en $C_1$-$C_{12}$, ou représente un radical phényle le cas échéant substitué une à quatre fois, de manière identique ou différente, par un atome d'halogène, un radical alcoyle en $C_1$-$C_{12}$, halogénoalcoyle en $C_1$-$C_{12}$, alcoxy en $C_1$-$C_{12}$ ou halogénoalcoxy en $C_1$-$C_{12}$ ;
où $R^1$ et $R^3$ ne représentent pas simultanément l'atome d'hydrogène.

**2.** Composés de la formule (I) suivant la revendication 1, dans laquelle :

$X^1$ représente l'atome d'hydrogène, de fluor ou de chlore ;
$X^2$ représente l'atome de fluor, de chlore, de brome, d'iode, un radical alcoyle en $C_1$-$C_3$ ou alcoxy en $C_1$-$C_3$ ;
$X^3$ représente l'atome d'hydrogène, de fluor, de chlore, un radical alcoyle en $C_1$-$C_3$ ou alcoxy en $C_1$-$C_3$ ;
$R^1$ représente l'atome d'hydrogène, de fluor, de chlore, de brome, un radical alcoyle en $C_1$-$C_4$, ou le radical $-CH_2-CR^4=CH_2$ ;
$R^2$ représente un radical alcoyle en $C_1$-$C_6$, hydroxyalcoyle en $C_1$-$C_4$, alcényle en $C_3$-$C_{10}$, alcynyle en $C_3$-$C_5$, un radical cyclohexyle ou cycloalcényle en $C_4$-$C_6$, chaque fois le cas échéant substitué par un atome d'halogène ou par un radical alcoyle en $C_1$-$C_4$, représente un radical (cycloalcoyl en $C_3$-$C_6$)alcoyle en $C_1$-$C_4$, le cas échéant substitué par un atome d'halogène, par un radical alcoyle en $C_1$-$C_4$, alcényle en $C_2$-$C_4$, halogénoalcényle en $C_2$-$C_3$, phényle, halogénophényle, styryle ou halogénostyryle, représente un radical (cycloalcényl en $C_4$-$C_6$)méthyle, le cas échéant substitué par un atome d'halogène, ou représente le radical benzyle, le cas échéant substitué une ou deux fois, de manière identique ou différente, par le radical nitro, un atome d'halogène, un radical alcoyle en $C_1$-$C_4$, halogénoalcoyle en $C_1$-$C_4$, alcoylthio en $C_1$-$C_4$, halogénoalcoylthio en $C_1$-$C_4$, alcoxy en $C_1$-$C_4$ ou halogénoalcoxy en $C_1$-$C_4$, représente le radical naphtylméthyle ou tétrahydronaphtylméthyle ;
$R^3$ représente l'atome d'hydrogène, un radical alcoyle en $C_1$-$C_3$, l'atome de chlore, de brome, ou le radical $-CH_2-CR^4=CH_2$ ;
$R^4$ représente l'atome d'hydrogène, un radical alcoyle en $C_1$-$C_4$, ou représente un radical phényle le cas échéant substitué une ou deux fois, de manière identique ou différente, par l'atome de fluor, de chlore, de brome, un radical alcoyle en $C_1$-$C_4$, halogénoalcoyle en $C_1$-$C_4$, ou halogénoalcoxy en $C_1$-$C_4$ ;

où $R^1$ et $R^3$ ne représentent pas simultanément l'atome d'hydrogène.

**3.** Composés de la formule (I) suivant la revendication 1, dans laquelle :

$X^1$ représente l'atome d'hydrogène, de fluor ou de chlore ;
$X^2$ représente l'atome de fluor, de chlore, de brome, d'iode, le radical méthyle ou méthoxy ;
$X^3$ représente l'atome d'hydrogène, de fluor, de chlore ou le radical méthyle ;
$R^1$ représente l'atome d'hydrogène, de fluor, de chlore, de brome, le radical méthyle, éthyle, n- ou i-propyle ou allyle ;
$R^2$ représente le radical méthyle, éthyle, n- ou i-propyle, n-butyle, i-butyle, s-butyle, n-pentyle, n-hexyle, cyclohexyle, hydroxyéthyle, hydroxypropyle, hydroxybutyle, $-CH_2-CH=CH_2$, $-CH_2-C(CH_3)=CH_2$, $-CH_2-C\equiv CH$, $-CH(CH_3)-C\equiv CH$, représente un des groupements cycloalcoylalcoyle :

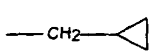 , 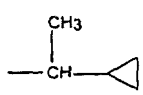 , 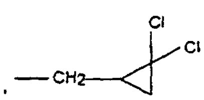 , 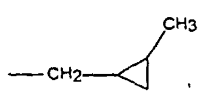 ,

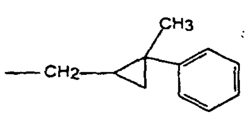 , 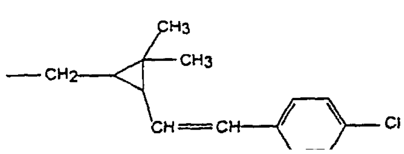 , 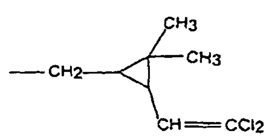 ,

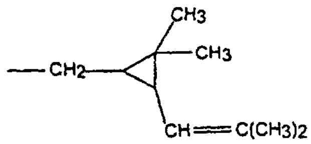 ;

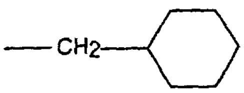 , 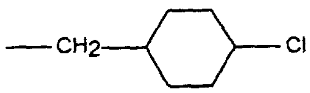 , 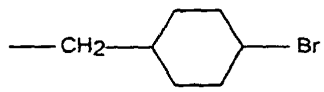 ,

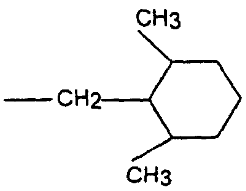 , 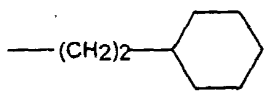 , 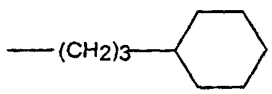 ,

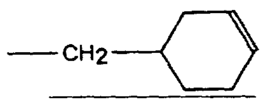 ,

représente l'un des restes suivants :

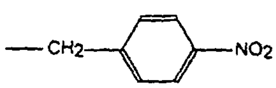 , 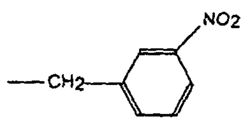 , 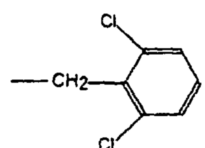 ,

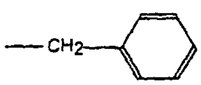 , 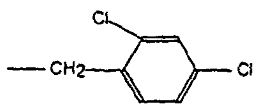 , 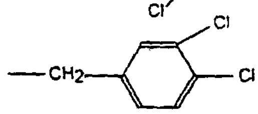 ,

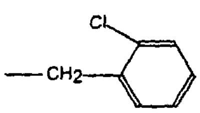 , 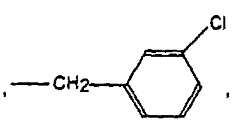 , 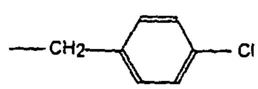 ,

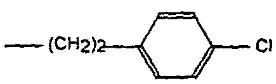 , 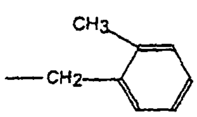 , 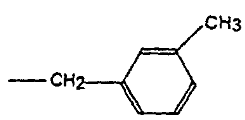 ,

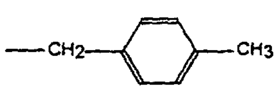 , 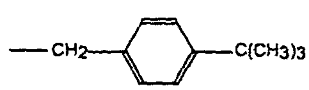 ,

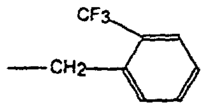 , 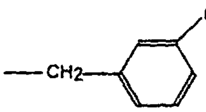 , 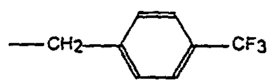 ,

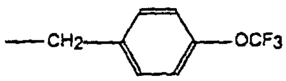 , 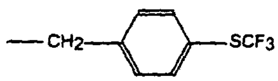 , 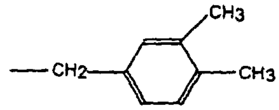

ou représente

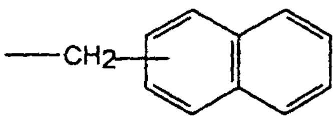 ou 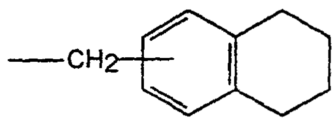

$R^3$ représente l'atome d'hydrogène, de chlore, de brome, le radical méthyle ou le radical $-CH_2-CH=CH_2$, en particulier l'atome d'hydrogène, de chlore ou le radical $-CH_2-CH=CH_2$, où $R^1$ et $R^3$ ne représentant pas simultanément l'atome d'hydrogène.

4. Procédé de préparation de composés de la formule (I) suivant les revendications 1 à 3 et de la formule (Ia) suivant les revendications 5 et 6, **caractérisé en ce que** :

A) on fait réagir des composés de la formule (II) :

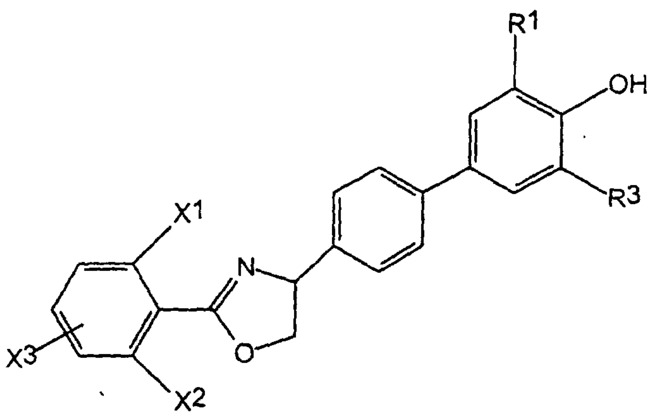 (II)

dans laquelle

$X^1$, $X^2$, $X^3$, $R^3$ et $R^1$ ont les significations indiquées à la revendication 1, où ici $R^1 = R^3$ = hydrogène est possible.

avec des composés de la formule (III) :

$$Z - R^2 \quad (III)$$

dans laquelle:

$R^2$ a la signification indiquée à la revendication 1, et
Z représente un groupe partant,

le cas échéant en présence d'un agent de dilution et le cas échéant en présence d'une base, et
B) le cas échéant, pour $R^2$ = $-CH_2-CR^4=CH_2$ et $R^3$ = H, on soumet ensuite les composés de la formule (IV) obtenus :

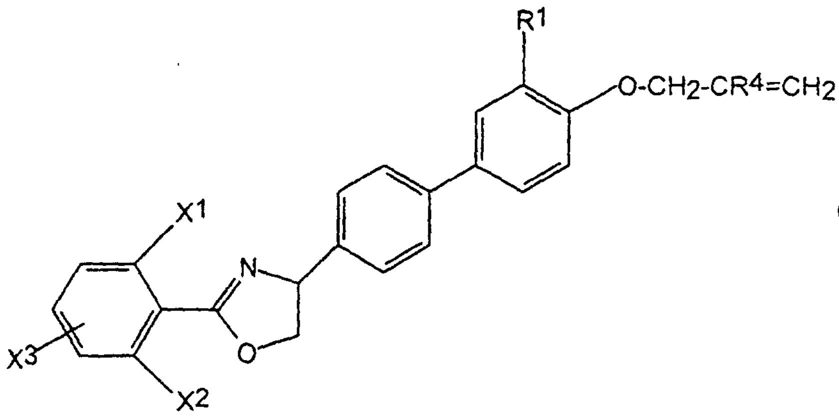

(IV)

dans laquelle :

$R^1$, $R^4$, $X^1$, $X^2$ et $X^3$ ont les significations indiquées à la revendication 1,

à une transposition de Claisen, et
C) le cas échéant, on fait réagir les composés de la formule (V) ainsi obtenus ;

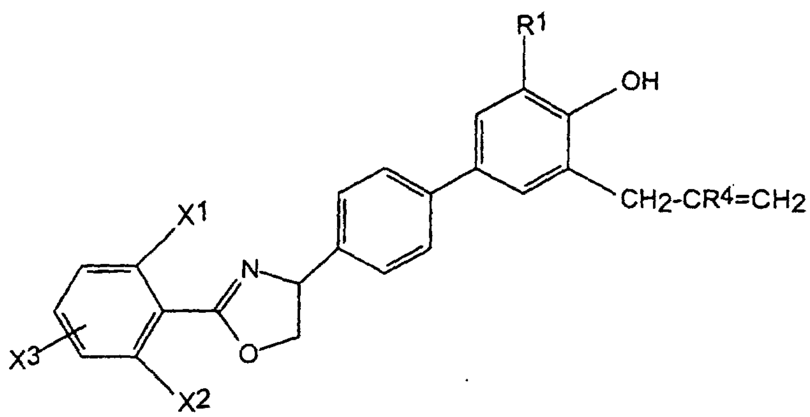

(V)

dans laquelle
$X^1$, $X^2$, $X^3$, $R^1$ et $R^4$ ont les significations indiquées à la revendication 1,
avec des composés de la formule (III) :

$$Z - R^2 \quad \text{(III)}$$

dans laquelle:

$R^2$ et Z ont les significations indiquées ci-dessus,
le cas échéant en présence d'un agent de dilution et le cas échéant en présence d'une base, et
le cas échéant pour $R^2$ = $-CH_2-CR^4=CH_2$ et $R^1$ = H, on répète les étapes B) et C), où on obtient des composés

de la formule (Va) :

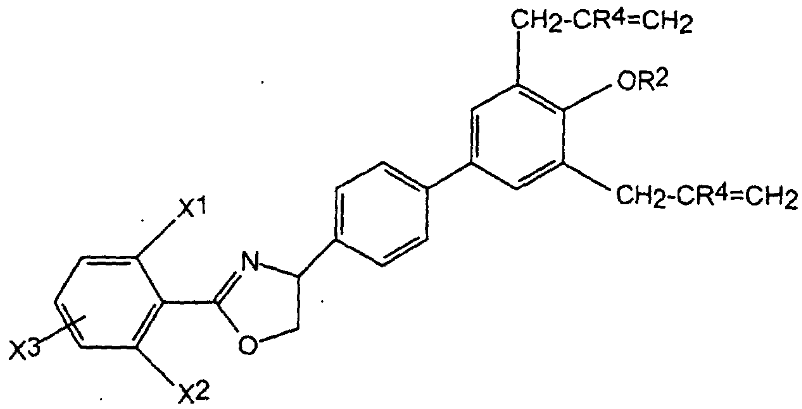

(Va)

dans laquelle
$X^1$, $X^2$, $X^3$, $R^2$ et $R^4$ ont les significations indiquées ci-dessus,
et les restes $R^4$ peuvent être identiques ou différentes.

**5.** Composés de la formule (Ia) :

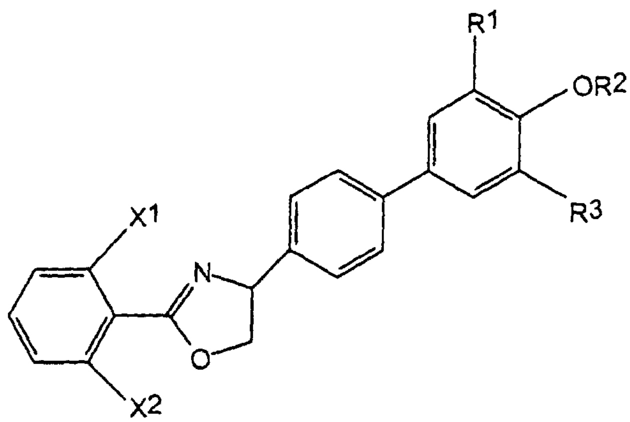

(Ia)

dans laquelle :

$R^1$, $R^2$, $R^3$, $X^1$, et $X^2$ ont les significations indiquées à la revendication 1.

**6.** Composés de la formule (Ia) suivant la revendication 5, dans laquelle :

$X^1$ représente l'atome d'hydrogène ou de fluor ;
$X^2$ représente l'atome de fluor, de chlore, de brome ou le radical méthyle ;
$R^1$ représente l'atome de chlore, le radical méthyle, éthyle, n- ou i-propyle ou allyle ;
$R^2$ a la signification indiquée à la revendication 1,
$R^3$ a la signification indiquée à la revendication 1.

**7.** Agent de lutte contre les parasites, **caractérisé par** une teneur en au moins un composé de la formule (I) suivant la revendication 1.

**8.** Utilisation des composés de la formule (I) suivant la revendication 1 pour lutter contre les parasites.

**9.** Procédé pour lutter contre les parasites, **caractérisé en ce que** l'on fait agir les composés de la formule (I) suivant la revendication 1 sur les parasites et/ou leur biotope.

**10.** Procédé de préparation d'un agent de lutte contre les parasites, **caractérisé en ce que** l'on mélange des composés de la formule (I) suivant la revendication 1 avec des diluants et/ou tensioactifs.

**11.** Utilisation des composés de la formule (I) suivant la revendication 1 pour la préparation d'agents de lutte contre les parasites.